# EUROPEAN PATENT SPECIFICATION

(11) **EP 2 307 568 B1**
(45) Date of publication and mention of the grant of the patent: **09.04.2014**
(21) Application number: 09769011.9
(22) Date of filing: 25.06.2009
(51) Int. Cl.: C12Q 1/68

(54) **GENOME WIDE VISUAL IDENTIFICATION OF HUMAN CO-FACTORS OF HIV-1 INFECTION**
GENOMWEITE OPTISCHE IDENTIFIZIERUNG MENSCHLICHER COFAKTOREN EINER HIV-1-INFEKTION
IDENTIFICATION VISUELLE SUR L'ENSEMBLE DU GÉNOME DE CO-FACTEURS HUMAINS DE L'INFECTION PAR LE VIH-1

(30) Priority: 25.06.2008 US 133027 P
(43) Date of publication of application: 13.04.2011
(73) Proprietor: Institut Pasteur Korea, Gyeonggi-do (KR); Institut Pasteur, 75724 Paris Cédex 15 (FR)
(72) Inventor: EMANS, Neil, Brooklyn, 0182 Pretoria (ZA); GENOVESIO, Auguste, 75013 Paris (FR); WINDISCH, Marc P., 45711 Datteln (DE); KWON, Yong Jun, Seongnam-si, Gyeonggi-do, 463-891 (KR); JUNG, Sungyong, Guro-gu, Seoul, 152-773 (KR); KIM, Hi Chul, Gygeongi-do 445-395 (KR); KIM, Nam Youl, Seoul (KR); CHOI, Seo Yeon, Gangdong-gu, Seoul, 134-793 (KR); NEHRBASS, Ulf, 67000 Strasbourg (FR)
(74) Representative: Engelhard, Markus
(86) International application number: PCT/EP2009/004604
(87) International publication number: WO 2009/156162

(56) References cited:
- WO-A-2007/141580
- WO-A2-2005/110338
- US-A1- 2004 248 145
- CROW YANICK J ET AL: "Mutations in genes encoding ribonuclease H2 subunits cause Aicardi-Goutieres syndrome and mimic congenital viral brain infection" NATURE GENETICS, NATURE PUBLISHING GROUP, NEW YORK, US, vol. 38, no. 8, 1 August 2006 (2006-08-01) , pages 910-916, XP002466050 ISSN: 1061-4036
- NOVINA C D ET AL: "siRNA directed inhibition of HIV-1 infection" NATURE MEDICINE, NATURE PUBLISHING GROUP, NEW YORK, NY, US, vol. 7, no. 8, 1 July 2002 (2002-07-01), pages 681-686, XP002955298 ISSN: 1078-8956
- WANINGER SHANI ET AL: "Identification of cellular cofactors for human immunodeficiency virus replication via a ribozyme-based genomics approach." JOURNAL OF VIROLOGY DEC 2004, vol. 78, no. 23, December 2004 (2004-12), pages 12829-12837, XP002541294 ISSN: 0022-538X
- BRASS ABRAHAM L ET AL: "Identification of host proteins required for HIV infection through a functional genomic screen" SCIENCE (WASHINGTON D C), vol. 319, no. 5865, February 2008 (2008-02), pages 921-926, XP002541295 ISSN: 0036-8075 cited in the application
- DATABASE EMBL [Online] 3 February 2004 (2004-02-03), "Sequence 6736 from Patent WO02068579." XP002562348 retrieved from EBI accession no. EMBL:CQ720802 Database accession no. CQ720802 & WO 02/068579 A2 (PE CORP NY [US]) 6 September 2002 (2002-09-06)
- DATABASE Geneseq [Online] 20 May 2004 (2004-05-20), "Human cDNA of the invention SEQ ID NO:1056." XP002562349 retrieved from EBI accession no. GSN:ADM02371 Database accession no. ADM02371 & EP 1 347 046 A1 (RES ASS FOR BIOTECHNOLOGY [JP]) 24 September 2003 (2003-09-24)
- DATABASE EMBL [Online] 18 December 2003 (2003-12-18), "Sequence 15 from Patent WO02068649." XP002562350 retrieved from EBI accession no. EMBL:AX921675 Database accession no. AX921675 & WO 02/068649 A2 (CURAGEN CORP [US]; TCHERNEV VELIZAR T [US]; SPYTEK KIMBERLY A [US]; ZE) 6 September 2002 (2002-09-06)
- DATABASE Geneseq [Online] 1 November 2007 (2007-11-01), "Human junction-mediating and regulatory protein (JMY), MCR cDNA." XP002562351 retrieved from EBI accession no. GSN:AJF46707 Database accession no. AJF46707 & WO 2007/095186 A2 (DANA FARBER CANCER INST INC [US]; DEPINHO RONALD A [US]) 23 August 2007 (2007-08-23)
- DATABASE EMBL [Online] 18 August 2004 (2004-08-18), "RZPD Homo sapiens cDNA clone IMAGp998H242004 = IMAGE:811847 5' EST." XP002562352 retrieved from EBI accession no. EMBL:CR747076 Database accession no. CR747076
- DATABASE Geneseq [Online] 20 May 2004 (2004-05-20), "Human cDNA of the invention SEQ ID NO:569." XP002562353 retrieved from EBI accession no. GSN:ADM01884 Database accession no. ADM01884 & EP 1 347 046 A1 (RES ASS FOR BIOTECHNOLOGY [JP]) 24 September 2003 (2003-09-24)

### SEQUENCE LISTING

<110> Institut Pasteur Korea
<120> Genome wide visual identification of human co-factors of HIV-1 infection
<130> I31628PCT
<160> 46
<170> PatentIn version 3.5
<210> 1
   <211> 2859
   <212> DNA
   <213> Homo sapiens
<400> 1
   attctggagt ccagagccac tgcctttgct ccagccgctg ccgccgcacc acctctcctt     60
   ctctgcctct gaccctcctt ctcgctgctc cccctgccca gctgctcctc ccacctggcc    120
   atgaccaaag cccctgctgg caccctggcc cagctctgag tcctgggacc ctcggtcctc    180
   tctcctgggc catggccaac tcaggcctcc agctcctggg ctacttcttg gccctgggtg    240
   gctgggtggg catcattgct agcacagccc tgccacagtg gaagcagtct tcctacgcag    300
   gcgacgccat catcactgcc gtgggcctct atgaagggct ctggatgtcc tgcgcctccc    360
   agagcactgg gcaagtgcag tgcaagctct acgactcgct gctcgccctg gacggtcaca    420
   tccaatcagc gcgggccctg atggtggtgg ccgtgctcct gggcttcgtg gccatggtcc    480
   tcagcgtagt tggcatgaag tgtacgcggg tgggagacag caaccccatt gccaagggcc    540
   gtgttgccat cgccggggga gccctcttca tcctggcagg cctctgcact ttgactgctg    600
   tctcgtggta tgccaccctg gtgacccagg agttcttcaa cccaagcaca cctgtcaatg    660
   ccaggtatga atttggccca gccctgttcg tgggctgggc ctcagctggc ctggccgtgc    720
   tgggcggctc cttcctctgc tgcacatgcc cggagccaga gagacccaac agcagcccac    780
   agccctatcg gcctggaccc tctgctgctg cccgagaacc agttgttaaa ttgcccgcct    840
   ccgccaaggg ccccctgggt gtgtaatgtc cagtccccag ccaggctctg tcccctgcca    900
   tacctagact gtgtgtttca tatttttttg gaaagagaag tgaacatcca gccccaatca    960
   tggtatcatt cggtctgtcc tcagcgtggc ttggacgggg cctgtgtcag agtggtcagt   1020
   gctgacccct ggggctcttg ggcagaaaga tgaggagaca gaggtccagg gtgggttaca   1080
   tagcacatcc agggctaagc aagaaataat tcagaggtcc taccctctgt ctagggaccc    1140
   ccctcccaag cctggccttg gccttggcac aaagtcctcc ttgataggag atcccactca   1200
   ctcctggagg ctgcccctga ggcttggccc agctctagga gcagtcccca gggtcaggga   1260
   gcccctggtg tggaaagagg ccccaaggta gtaaaccctg cccctgttac tgtgctccag   1320
   agacctccta agggaaggga cagttcctgg aaggccctcc agctggatgc tggggatcag 1380
   cgataggtga ggggacacag tgtaggagct ccccatgtag aaaagggaat gtggggaggg 1440
   cgttaggagc ttgcaggcat taggactgtc ctgagcaagg tctgcagccc ccagctctgc 1500
   tcaccccgaa tcctgcccct tgtttccaca cctaccattc ctcctctcct gatccccagc      1560
   atccagctga ggtccaaggt ctttgtccta gaatcagagt ggggagggga cagcctgggg      1620
   ctgcccagag actgtgggtg gagctgcctg ctgcactcag cagtgcggtc agagaagggc 1680
   ttttggtctt gaagtccagg taccatcccc ccttagcata cagggggaag ggcctgagag 1740
   gaatgtaagg aaaccagccc agatcagtcc caaggccaga gtcctttgtc ctacatctcc 1800
   ctgaaccaga gtgtgccctg cccctcatgc tcagacctct cccaccccaa accctctccc 1860
   gggactcagt ctccctggcc actgcgtatc aggcttctgg ggaaagcatc catcacagaa 1920
   cctccccttc cctgccacgc accttccttg gccagctcca ttctggcctc ctccaccacc 1980
   tgccttgtga ccacatctcc caccacgtcc ccagatctca agaacgcagc tcagcttctc 2040
   cttcgagctt gactctgaga gggaaagtga cggaaaccaa gtcagatgag atgactgcca      2100
   tgtacactgc agtcaagggc agggagggga ggaatgacac aaatggcagg gagctgctgg      2160
   gggactgacc cctcggcgcc tggcctggcc ggtgctgcac atccaccggg gcacaacagg 2220
   gacttgtcca gcctctggtc agaggatgtg gccacctgac cctaaatagg ttccccagag 2280
   tcctgcccct ctaatgaatg agaactgcag gagtttctcc tctgggtgcc tgaagctata 2340
   gtgcaatggt tcccaaccct gcatgcacat tcgaatcacc tgggggcaca atgcctaggc 2400
   tccaacccca gacactctta tttcattggt ctgggtggac ctggcatcag aagtcatgta 2460
   gctcctcagg ggactgtagt gtgtggtcag cactgagggc tcctctatga ggcctcaagc 2520
   ccaggtgact ctgtgaggtc tgcagaggga gaaaagaacc cacaagggaa gaggtggagg 2580
   tcaggcacgg tggctcacgc ttgtaatccc agcactttgg gaggccgagg tgggtagata 2640
   cctgaagtca ggagttcgag actagcctgg ccaatatggt aaaaccccgt gtctattaaa 2700
   aatacaaaaa ttagctggct gtggtggtgg gcacctgtaa tcccagctac tcgggaggct 2760
   gaggcaggag aatcgcttga actcgggagg tggaggttgc agtcagccaa gatcgtgcca 2820
   ctgcacacca tcctggatga cagagcaaga ctccatcac 2859
<210> 2
   <211> 3602
   <212> DNA
   <213> Homo sapiens
<400> 2
   attctggagt ccagagccac tgcctttgct ccagccgctg ccgccgcacc acctctcctt 60
   ctctgcctct gaccctcctt ctcgctgctc cccctgccca gctgctcctc ccacctggcc 120
   atgaccaaag cccctgctgg caccctggcc cagctctgag tcctgggacc ctcggtcctc 180
   tctcctgggc catggccaac tcaggcctcc agctcctggg ctacttcttg gccctgggtg 240
   gctgggtggg catcattgct agcacagccc tgccacagtg gaagcagtct tcctacgcag 300
   gcgacgccat catcactgcc gtgggcctct atgaagggct ctggatgtcc tgcgcctccc 360
   agagcactgg gcaagtgcag tgcaagctct acgactcgct gctcgccctg gacggtcaca 420
   tccaatcagc gcgggccctg atggtggtgg ccgtgctcct gggcttcgtg gccatggtcc 480
   tcagcgtagt tggcatgaag tgtacgcggg tgggagacag caaccccatt gccaagggcc 540
   gtgttgccat cgccggggga gccctcttca tcctggcagg cctctgcact ttgactgctg 600
   tctcgtggta tgccaccctg gtgacccagg agttcttcaa cccaagcaca cctgtcaatg 660
   ccaggtatga atttggccca gccctgttcg tgggctgggc ctcagctggc ctggccgtgc 720
   tgggcggctc cttcctctgc tgcacatgcc cggagccaga gagacccaac agcagcccac 780
   agccctatcg gcctggaccc tctgctgctg cccgagagta cgtctgagct ccgcctgccc 840
   tggccagccc cccacccagt ggcccccttg cccagcatcc agccagcctc gcagcaccct 900
   gggcagggcc actggggcat aggatgggca taggtgctct gagcagcttg tcctcaacac 960
   aagcacccac cctgcaatct gagacccaga tcctcagaga gacaccagag gcaggaccca 1020
   gcccccaggc atacacacag atgcaggtcc aggcacggtc ttgtctgcac agcctggtgg 1080
   gcaccagcat gcatccctgg agacaggccc tcaggcacca gcccggctgt ttactcactg 1140
   aagagctgct tgggtgtctg ctacgtgctg ggccctagag atagagcagt ggccaagacg 1200
   taccttagta cccaggtcct tggggtgagc agaaaccttc accctcccca gtcccatggg 1260
   ctcctcacag caaccccaca agggcagtgc cgggatgctg aacgttcaca caaggacagg 1320
   gagggtctga gtttaggtct caggttcttc cagtgcgccc agggctgggg gccacctaca 1380
   cagatggtga ggtcggacca tggcgcccct gcccccggga atgggcccca ggcagggctg 1440
   ctgtgagggc caaggtctgg ccacgctggc cagtacccat gtccgggcct gaatgcacag 1500
   cccctgcccc cgaccccaca gctcactcca ctaaccagct ctctctcttt tgactttcag 1560
   accagttgtt aaattgcccg cctccgccaa gggccccctg ggtgtgtaat gtccagtccc 1620
   cagccaggct ctgtcccctg ccatacctag actgtgtgtt tcatattttt ttggaaagag 1680
   aagtgaacat ccagccccaa tcatggtatc attcggtctg tcctcagcgt ggcttggacg 1740
   gggcctgtgt cagagtggtc agtgctgacc cctggggctc ttgggcagaa agatgaggag 1800
   acagaggtcc agggtgggtt acatagcaca tccagggcta agcaagaaat aattcagagg 1860
   tcctaccctc tgtctaggga cccccctccc aagcctggcc ttggccttgg cacaaagtcc 1920
   tccttgatag gagatcccac tcactcctgg aggctgcccc tgaggcttgg cccagctcta 1980
   ggagcagtcc ccagggtcag ggagcccctg gtgtggaaag aggccccaag gtagtaaacc 2040
   ctgcccctgt tactgtgctc cagagacctc ctaagggaag ggacagttcc tggaaggccc 2100
   tccagctgga tgctggggat cagcgatagg tgaggggaca cagtgtagga gctccccatg 2160
   tagaaaaggg aatgtgggga gggcgttagg agcttgcagg cattaggact gtcctgagca 2220
   aggtctgcag cccccagctc tgctcacccc gaatcctgcc ccttgtttcc acacctacca 2280
   ttcctcctct cctgatcccc agcatccagc tgaggtccaa ggtctttgtc ctagaatcag 2340
   agtggggagg ggacagcctg gggctgccca gagactgtgg gtggagctgc ctgctgcact    2400
   cagcagtgcg gtcagagaag ggcttttggt cttgaagtcc aggtaccatc cccccttagc    2460
   atacaggggg aagggcctga gaggaatgta aggaaaccag cccagatcag tcccaaggcc 2520
   agagtccttt gtcctacatc tccctgaacc agagtgtgcc ctgcccctca tgctcagacc 2580
   tctcccaccc caaaccctct cccgggactc agtctccctg gccactgcgt atcaggcttc 2640
   tggggaaagc atccatcaca gaacctcccc ttccctgcca cgcaccttcc ttggccagct 2700
   ccattctggc ctcctccacc acctgccttg tgaccacatc tcccaccacg tccccagatc 2760
   tcaagaacgc agctcagctt ctccttcgag cttgactctg agagggaaag tgacggaaac 2820
   caagtcagat gagatgactg ccatgtacac tgcagtcaag ggcagggagg ggaggaatga 2880
   cacaaatggc agggagctgc tgggggactg acccctcggc gcctggcctg gccggtgctg 2940
   cacatccacc ggggcacaac agggacttgt ccagcctctg gtcagaggat gtggccacct 3000
   gaccctaaat aggttcccca gagtcctgcc cctctaatga atgagaactg caggagtttc 3060
   tcctctgggt gcctgaagct atagtgcaat ggttcccaac cctgcatgca cattcgaatc 3120
   acctgggggc acaatgccta ggctccaacc ccagacactc ttatttcatt ggtctgggtg 3180
   gacctggcat cagaagtcat gtagctcctc aggggactgt agtgtgtggt cagcactgag 3240
   ggctcctcta tgaggcctca agcccaggtg actctgtgag gtctgcagag ggagaaaaga 3300
   acccacaagg gaagaggtgg aggtcaggca cggtggctca cgcttgtaat cccagcactt 3360
   tgggaggccg aggtgggtag atacctgaag tcaggagttc gagactagcc tggccaatat 3420
   ggtaaaaccc cgtgtctatt aaaaatacaa aaattagctg gctgtggtgg tgggcacctg 3480
   taatcccagc tactcgggag gctgaggcag gagaatcgct tgaactcggg aggtggaggt 3540
   tgcagtcagc caagatcgtg ccactgcaca ccatcctgga tgacagagca agactccatc 3600
   ac 3602
<210> 3
   <211> 3262
   <212> DNA
   <213> Homo sapiens
<400> 3
   agcggttccg ggcggcagca caaggcggta gccatggcgg aggcggcggc tgcagcgggt 60
   gggactggct tgggcgcggg cgcgagctac gggtctgcag cggaccggga ccgggacccg 120
   gacccggacc gcgccgggcg gaggctgcgg gttctctctg gccatctgct gggccggccc 180
   cgggaggctc tgagtaccaa tgagtgcaaa gcgcggagag ccgcgtcggc ggccacggca 240
   gcgcccacgg ccactcccgc cgcgcaggag tcgggcacca tcccaaagaa gcggcaagaa 300
   gttatgaaat ggaatggatg gggatataat gattctaaat tcatcttcaa taagaagggc 360
   caaattgaat tgactgggaa aaggtaccct cttagtggca tgggtttacc aacatttaaa 420
   gaatggatcc aaaataccct tggagtaaat gtggagcata aaactacctc taaagcatcc 480
   ttaaatccta gtgatacacc tccttctgtt gtaaatgaag attttcttca tgaccttaaa 540
   gaaactaata tttcatattc acaagaggca gatgatcgag tatttagagc tcatggtcat 600
   tgtcttcatg agatattttt gctcagggaa ggaatgtttg agcgaattcc tgatatagtt 660
   ttatggccaa catgccatga tgatgtagtt aagattgtga atctagcttg caaatataat 720
   ctttgtatca taccaattgg tggaggaaca agtgtttcat atggcctgat gtgtcctgca 780
   gatgagacaa gaacaattat ttctttggac acttcacaaa tgaatcgaat tctctgggtt 840
   gatgagaaca atttgacagc tcatgtagag gctggcataa caggacaaga gttggaaaga 900
   cagcttaaag aaagtggtta ttgtacaggt catgaaccag attccctgga gttcagtact 960
   gtaggaggat gggtatctac tcgcgcatca ggcatgaaga agaatatcta tggcaatatc 1020
   gaggacctgg tggttcatat aaaaatggta acacctagag gtataataga aaaaagctgt 1080
   caaggacctc gtatgtcaac aggccctgat atccatcact tcatcatggg atctgaagga 1140
   actcttggtg taataacaga agctacaata aaaatcagac cagtccctga ataccaaaag 1200
   tatggctcag tagctttccc taattttgaa caaggagtag cctgtttaag agaaattgca 1260
   aaacagagat gtgctccggc atctattcgc ctcatggaca acaagcagtt tcagtttggt 1320
   catgctctta aacctcaggt ttcctctatt tttacatcat ttttggacgg attaaaaaag 1380
   ttttatatta caaagtttaa aggatttgac ccaaatcagc taagtgtagc cacattactg 1440
   tttgaggggg atcgtgagaa ggttcttcaa catgaaaaac aagtgtatga tattgctgca 1500
   aaatttggtg ggttggcagc tggagaagat aatggacaga gaggttattt gctgacctat 1560
   gttattgcat acattcgaga cttggctttg gaatactatg tattaggaga atcttttgag 1620
   acttctgctc cttgggacag ggtggtagat ctctgtagaa atgtaaaaga aagaataaca 1680
   agggaatgca aagagaaggg tgttcagttt gctccttttt ctacatgcag ggtgacgcag 1740
   acttacgatg caggtgcttg tatctacttc tattttgcct ttaactacag gggaattagt 1800
   gacccactga ccgtatttga acaaactgag gcagctgcta gagaagaaat ccttgctaat 1860
   ggagggagcc tgtcacatca ccatggagtg ggcaagttac ggaagcaatg gctaaaggaa 1920
   agtatctctg atgtcggctt tgggatgctg aagtctgtca aggaatatgt ggaccccaat 1980
   aacatctttg gaaacagaaa ccttttataa atccattagt accattacaa aaaaatgtca 2040
   attttttttt taagttttca actgtggtta tactagtaat caaatatatc atggactata 2100
   ttttggatac atttgtttct ttggtttaaa ataagtttgt tttcattctg tagtttgttt 2160
   tgtttctaca tctatggatt gacagatagt attcctaaat ctctctcatt gtaggtacat 2220
   cattttacat tcagcggttg tcatttcaaa ttttagtcag gcagcacaaa gctgtcaatt 2280
   attccagagg aagctgctgc cagctgtttt gtattgttgc ttcctcttgg ggaacaaaga 2340
   cagatttggt atcattgatt gctcagctag cctcttttta aaagaacgtt tctgggaatc 2400
   agatgtctag ggaaggattc cactgaggag tgatacacgt gtacattgtt taagagcata 2460
   gtctagtgtg agtaggccat taaggggaag gatattcatg aaagaattta ggcaacctgt 2520
   tatttagaaa gccttcacgt ctgtaaggtg cttggcctac tttttttttt aaattagtat 2580
   acatgcataa aaccttgtat catcaatata caaattattc tgttagggga catgtttttg 2640
   ttctccatgt ttctgtttct attgtggtat gagttgtatt tgtatcactg agataaccta 2700
   ttgttatatt ctaattcatt gataatatgt tttgtaagaa aagctgacat ccttcattgc 2760
   aagtgaagaa caggtttacc atgaaatgaa tgcctttatt caacttagag tcctaatgtt 2820
   tctgatttca cattcttgtt gctgaaatgc agaagaaaca gctacagcaa gagcagaagg 2880
   aaaactctta gacttaatgt ctccaattgc aagaattgtt tcactaaaga aacagtcatc 2940
   attcaactac tgaatttgaa agcctcagca gtttcaatga caaaaatcat ttttgatttt 3000
   ttaaaattaa tcccaatggc atgataaatt ttcatataaa aagtaaagtt tcaatttagt 3060
   ttttaataat ggtttaatgc cttttttgaa taactggttt aacctaattt tttttaaatg 3120
   taatgtatta atgcatatac cataatcaaa agtttgaaat atcctgtttc ttaaataatg 3180
   agaacattaa tcacatttag caagcatttg tacattcaaa cctggatatt aaagtgaaat 3240
   gattactttg aaaaaaaaaa aa 3262
<210> 4
   <211> 2837
   <212> DNA
   <213> Homo sapiens
<400> 4
   agtctcgcgg gaagctccgt tgtgggcgcc ccggctggtg gctgagctca ggccttcagg 60
   cagaggggag gcgagggcgg ggcggtcacg tgagagcact gccgcggtgg gttgtggggg 120
   tgctgcggcg ccgtttgctt tgccaaaccg acaaaagaga gatgatggcc aacgacgcca 180
   agcccgacgt gaagaccgtg caggtgctgc gggacacagc caaccgcctg cggatccatt 240
   ccatcagggc cacgtgtgcc tctggttctg gccagctcac gtcgtgctgc agtgcagcgg 300
   aggtcgtgtc tgtcctcttc ttccacacga tgaagtataa acagacagac ccagaacacc    360
   cggacaacga ccggttcatc ctctccaggg gacatgctgc tcctatcctc tatgctgctt    420
   gggtggaggt gggtgacatc agtgaatctg acttgctgaa cctgaggaaa cttcacagcg    480
   acttggagag acaccctacc ccccgattgc cgtttgttga cgtggcaaca gggtccctag    540
   gtcagggatt aggtactgca tgtggaatgg cttatactgg caagtacctt gacaaggcca    600
   gctaccgggt gttctgcctt atgggagatg gcgaatcctc agaaggctct gtgtgggagg    660
   cttttgcttt tgcctcccac tacaacttgg acaatctcgt ggcggtcttc gacgtgaacc     720
   gcttgggaca aagtggccct gcaccccttg agcatggcgc agacatctac cagaattgct     780
   gtgaagcctt tggatggaat acttacttag tggatggcca tgatgtggag gccttgtgcc    840
   aagcattttg gcaagcaagt caagtgaaga acaagcctac tgctatagtt gccaagacct    900
   tcaaaggtcg gggtattcca aatattgagg atgcagaaaa ttggcatgga aagccagtgc    960
   caaaagaaag agcagatgca attgtcaaat taattgagag tcagatacag accaatgaga    1020
   atctcatacc aaaatcgcct gtggaagact cacctcaaat aagcatcaca gatataaaaa    1080
   tgacctcccc acctgcttac aaagttggtg acaagatagc tactcagaaa acatatggtt    1140
   tggctctggc taaactgggc cgtgcaaatg aaagagttat tgttctgagt ggtgacacga    1200
   tgaactccac cttttctgag atattcagga aagaacaccc tgagcgtttc atagagtgta    1260
   ttattgctga acaaaacatg gtaagtgtgg cactaggctg tgctacacgt ggtcgaacca    1320
   ttgcttttgc tggtgctttt gctgcctttt ttactagagc attcgatcag ctccgaatgg    1380
   gagccatttc tcaagccaat atcaacctta ttggttccca ctgtggggta tccactggag    1440
   aagatggagt ctcccagatg gccctggagg atctagccat gttccgaagc attcccaatt    1500
   gtactgtttt ctatccaagt gatgccatct cgacagagca tgctatttat ctagccgcca    1560
   ataccaaggg aatgtgcttc attcgaacca gccaaccaga aactgcagtt atttataccc    1620
   cacaagaaaa ttttgagatt ggccaggcca aggtggtccg ccacggtgtc aatgataaag    1680
   tcacagtaat tggagctgga gttactctcc atgaagcctt agaagctgct gaccatcttt    1740
   ctcaacaagg tatttctgtc cgtgtcatcg acccatttac cattaaaccc ctggatgccg    1800
   ccaccatcat ctccagtgca aaagccacag gcggccgagt tatcacagtg gaggatcact    1860
   acagggaagg tggcattgga gaagctgttt gtgcagctgt ctccagggag cctgatatcc    1920
   ttgttcatca actggcagtg tcaggagtgc ctcaacgtgg gaaaactagt gaattgctgg    1980
   atatgtttgg aatcagtacc agacacatta tagcagccgt aacacttact ttaatgaagt    2040
   aaactaggct tatttctaaa aagtcaagtc tattggcttt ggcccaaaag cactggtatc    2100
   tttgtattaa attcatgttt attgtcacaa aaccattatt tatacctata cagttgtact    2160
   gtttctttta aagcaaagcc atttaacatc tttcttcatt cctaatttgg aaattaaagt    2220
   ttacctttct gttaatctat gtataaatgt tactctgagt tattaatgtg gattttaaaa 2280
   ttgtaagcaa tagaatagga aataaaacaa ctacctaata caaatatttc tgataagact 2340
   acaaatatct gactgagctg gggattaaag tagaggtaac tgtatcttaa atgagtatga 2400
   tttccttgta agttaaaaaa attgaaattt aattgtagac ttcaatagtc caagttttga 2460
   aggatgtttg agcttttgta taatgccatt tatacctgca gttttacaga taatgtttga 2520
   ctgcagttgc cttggaaatt cctccaaagt ttgccttcat ctctcctcta cagtttggag 2580
   gtgatggtgc agcagtggaa catctcttga tgcaccacac tacttgtgtt ctgtgaagtg 2640
   atgaaagtat aactggttct agtttgcaca ctacacacat agttttgtga agcttcagaa 2700
   atgttttttc ttttccttgt ggccaaacca gtttgttaat ctgattatat tcatctgcta 2760
   atgatactaa agttaatgta ataaagcatt taaaaatcag aaaaaaaaaa aaaaaaaaaa 2820
   aaaaaaaaaa aaaaaaa 2837
<210> 5
   <211> 2677
   <212> DNA
   <213> Homo sapiens
<400> 5
   gggggcgccc ccgagatgac cgagcaggaa atcgacactc tgtgttacca gctccaggtc 60
   tacctgggcc acggcctgga cacctgcggc tggaagatcc tctcccaggt gctcttcacc 120
   gagaccgatg atcccgagga gtattacgaa agcctcagcg agctgcggca gaagggctac 180
   gaagaagtgc ttcagcgggc caggaagcgc atccaggagc tcttggataa gcacaagaat 240
   acagagagca tggtggagct tctggacttg tatcagatgg aggatgaagc ctacagcagc 300
   cttgcagaag ctacaaccga actctatcag tatttactac agccattccg agacatgaga 360
   gaacttgcca tgctacgaag acagcagatc aagatttcca tggagaatga ttatctggga 420
   cctcgaagaa ttgagagtct acaaaaagaa gatgctgatt ggcagcggaa agctcacatg 480
   gctgtactgt ctattcaaga tcttactgtc aagtactttg aaataacagc taaagctcaa 540
   aaagctgtgt atgatcgaat gcgagctgat cagaagaaat ttggtaaagc atcatgggca 600
   gcggctgctg aacggatgga aaaactccag tatgcagttt ctaaggaaac tttgcagatg 660
   atgagagcta aagagatatg cttggaacag cggaaacatg cactaaagga agagatgcag 720
   agtttgcggg gtggtacaga agcgatagca cgattggatc agttagaagc tgattattat 780
   gatctgcaac ttcagttgta tgaagtacag tttgaaatct tgaagtgtga agagttacta 840
   ttgacagcgc aactagaaag catcaaaaga cttatatcag aaaaaagaga tgaagtggta 900
   tactatgaca cttacgaaag catggaggcc atgctggaga aggaagagat ggcagcatct 960
   gcgtacttac agagagaaga gctgcagaaa cttcagcaga aagcacgcca gctggaagca 1020
   agacgtggac gggtttctgc caagaaatcc tacctcagaa ataaaaagga aatatgtatt 1080
   gcaaaacaca atgaaaaaat ccaacagcgc actcggattg aagatgaata tagaacccat 1140
   cacacagtac aactaaagag agaaaaatta catgatgaag aagaaagaaa aagtgcctgg 1200
   gttagccaag agagacagag aacactggat agacttcgaa catttaaaca gaggtatcct 1260
   gggcaagtca tacttaaatc aaccagatta cgactagctc atgcaagaag aaaaggtgca 1320
   gcaagtcctg ttctccaaga ggatcattgt gactctttac caagtgtgtt acaggtagaa 1380
   gagaaaactg aagaggtggg agaaggaaga gtcaagcgtg ggccatcaca gacaacagaa 1440
   ccccagagcc ttgtgcaact tgaagatact tcattaacac aacttgaagc cacctcatta 1500
   cctctcagtg gtgttacctc tgaactgcct cccactatat ctcttccact tttgaataac 1560
   aacctcgaac catgttctgt taccataaat ccactcccat cccctcttcc tccaacacca 1620
   ccacctcccc cacctcctcc ccctccccca ccaccaccac ctctgcctgt tgctaaggac 1680
   agtggcccag agacactgga gaaagatctg cctagaaagg aggggaatga gaagaggatc 1740
   ccaaagtcag ccagtgcccc ctcagcacac ctctttgaca gcagccagct ggtcagtgca 1800
   cggaagaagc tcagaaagac tgctgaaggt ttgcagagga ggagagtgag ctcacccatg 1860
   gatgaggtgc tagcctcctt gaagcgtggt agttttcatc tgaaaaaggt tgaacagcga 1920
   actctgcctc cttttcctga tgaagatgat agtaataata tcttggcaca aataaggaaa 1980
   ggggtaaaat tgaagaaggt acagaaggat gttttgagag aatccttcac acttctaccc 2040
   gatacagacc ctctaacacg gagcatccat gaagctctta gaagaattaa agaagcatcc 2100
   ccagagtcag aggacgaaga ggaggcttta ccttgcacag actgggagaa ctaacaagtg 2160
   acataacaga agaaaaatat cattcaagat tggttctgat tcttttggaa aatgacagtt 2220
   taagcacttg gttcctcagt tggataacat tagatccaaa gttcattggt tcagtggtgt 2280
   gaagaaagga agcacaattg gcaggttatc actttccagt cgttccaata gatgatggtt 2340
   aacatgaatt ttacatgtgc aatgttcctg actgcaatga agaaaaggcc ttctggagat 2400
   ttctgttttt taagcacatt ctccttccat ccacatattt acatccttgt aatggccagt 2460
   taagctaggg cttgctgtaa gttggaagaa cactgggttg acagagatct actgtgagct 2520
   gtattgggac tgctttgaga accatctttc aatgcactga aaagtcatct gaaaaaatag 2580
   cttcttccat atcagcttat attaaatact gtgacactct gagaaagttt atcatcatga 2640
   tagctgtaag tcaggcatta aaatcaaatg gaaatac 2677
<210> 6
   <211> 8449
   <212> DNA
   <213> Homo sapiens
<400> 6
   gcgacctccg gcgccatttt gtagagaaac aagcggagtt aaccgaagag ggggtcgagg      60
   agagccggag tcggggaccc aggagtttcc tgtgtccagc gctgccggag ccgcctgagg     120
   tgccatgttt cagaacagag taagacccct ggtaaagaag aactgaagat attatacaga     180
   taccagatat agcctaatta caaagaaagc attaacctgc ctctgaggtg actaaagggg     240
   aataatggtg attttgcgcc gggctcggcc gcctgcttcc gccccaacca gcaatgaatc     300
   ttgactcgct ctcgctggcc ttgtctcaaa tcagctacct ggtggacaat ttaaccaaga     360
   aaaattaccg agccagccag caggaaatac agcatattgt gaatcggcac ggtcctgagg     420
   cagacaggca tttattacgc tgcctatttt cgcatgtgga tttcagtggc gatggtaaaa     480
   gcagtggcaa agatttccat cagactcagt ttctgattca ggagtgtgcg ttgctgatta     540
   caaagccaaa ttttatctcg acgctgtcct atgccattga taatccattg cactatcaga     600
   agagtttaaa gcctgcaccc cacttatttg cccagctgag taaagtgctc aaattaagca     660
   aagtacaaga ggtaattttt ggccttgccc tgttgaattc ttccagctca gatcttagag     720
   gtttcgctgc ccagtttatc aaacagaagc ttccagatct tctgcgttct tacattgacg     780
   cagacgtcag tggaaatcaa gaaggtggct tccaagatat agcaatagag gtcctacacc     840
   tcctcctctc ccatctcctc tttgggcaga agggagcctt tggagttgga caagaacaga     900
   tagacgcttt tcttaagacg ctgcgcagag attttcccca agaacgctgt cccgtggtgc     960
   tcgcaccact tttataccct gaaaaacggg acattctaat ggacaggatc ctgcctgatt    1020
   ccggaggggt agctaaaacc atgatggaga gctctttggc tgatttcatg caagaagtag    1080
   gctatggctt ttgtgcaagt attgaagaat gtcgcaatat aatcgtgcag tttggtgttc    1140
   gggaggtcac agctgcccag gttgcaaggg ttttgggaat gatggctcga actcattcag    1200
   gattaacaga tggcattcca ttacagagta tttctgctcc gggcagtggg atctggagtg    1260
   atgggaaaga taaaagtgat ggagcacagg cacacacatg gaatgtagaa gtcttgattg    1320
   acgttcttaa agaactgaat ccaagtttga atttcaagga agtaacttat gaactggacc    1380
   atcctggatt tcaaattcgt gacagtaaag gacttcataa tgtggtttat ggcattcaga    1440
   ggggtttggg tatggaagtg ttcccagtag acctcatata tagaccttgg aaacatgctg    1500
   aaggccagct ctccttcatt caacattccc ttataaatcc agagatcttc tgttttgctg    1560
   actatccctg tcatactgtt gccactgata ttctgaaagc accaccagag gatgacaatc    1620
   gagaaattgc cacatggaag agcttggatt tgattgaatc tctgctgagg cttgcagagg    1680
   ttgggcagta tgagcaagtc aaacagctct tcagcttccc tatcaaacac tgtccagaca    1740
   tgctggtatt ggccttacta caaattaaca cctcttggca taccttgcgc catgaactta    1800
   tctccactct gatgccaatt ttccttggaa accatcctaa ctcagctatt attttgcact    1860
   atgcatggca tgggcaggga cagtctccct caattcgcca acttatcatg catgcaatgg 1920
   cagaatggta catgagaggg gagcagtatg atcaggccaa attgtctcga atacttgatg 1980
   tggcccagga cttgaaggcc ttgtcaatgc tgctaaatgg tactccattt gcctttgtta 2040
   ttgaccttgc tgcacttgct tcacgtcgtg aatacctcaa acttgataag tggctcacag 2100
   ataaaattcg agagcatggg gagcctttta tccaggcgtg tatgactttt ttaaagagac 2160
   ggtgtccttc tattttgggc ggacttgccc cagaaaaaga ccagcccaaa agtgctcaac 2220
   ttcctccaga aactttggcg acaatgttgg cctgtctgca agcttgtgca gggagtgttt 2280
   ctcaggagct atcagaaact atcctcacca tggtagccaa ttgcagtaat gttatgaata 2340
   aggccagaca accaccacct ggagttatgc caaaaggacg tcctcctagt gctagcagct 2400
   tagatgccat ttctcctgtt cagattgacc ctcttgctgg aatgacatct cttagtatag 2460
   gtggttcagc tgcccctcac acccagagta tgcagggttt tcctccaaat ttgggttctg 2520
   cattcagtac ccctcagtca ccagcaaaag catttccacc cctttcaacc cccaatcaga 2580
   ccactgcatt cagtggtatt ggaggacttt catcacagct tccagtaggt ggtcttggca 2640
   caggcagcct gactggtata ggaactggtg ctcttggact ccctgcagtg aataacgacc 2700
   cttttgtaca gaggaaactg ggcacctctg gactgaatca gcctacattc cagcagagta 2760
   agatgaaacc ttcggacttg tctcaggtgt ggccagaggc aaaccagcac tttagtaaag 2820
   agatagatga tgaagcaaac agctatttcc agcgaatata taatcatcca ccacatccaa 2880
   ccatgtctgt tgatgaggta ttagaaatgc tgcagagatt taaagactct actataaaga 2940
   gggaacgaga agtatttaac tgtatgctaa ggaacttgtt tgaagaatat cgtttttttc 3000
   cccagtatcc tgataaagag ttacatataa cagcctgcct atttggtggt ataattgaga 3060
   aaggactggt cacttacatg gcactaggtc tggctctacg atatgttctt gaagccttac 3120
   gcaagccttt tggatccaaa atgtattatt tcgggattgc tgcactagat agatttaaaa 3180
   acagattgaa ggactatccc cagtattgtc agcatttggc ttctatcagt cactttatgc 3240
   aatttccaca tcatttacag gagtatattg agtatggaca gcagtctaga gatcctcctg 3300
   tgaaaatgca aggctctatc acaacccctg gaagtattgc actggctcag gcccaggctc 3360
   aggcccaggt tccagcaaaa gctcctcttg ctggtcaagt tagcactatg gtaaccacct 3420
   caacaactac cactgttgct aaaacggtta cggtcaccag gccaactgga gtcagcttta 3480
   agaaagatgt gccaccttct attaatacta caaatataga tacgttgctt gtggccacag 3540
   atcaaactga gagaattgtg gagcccccag aaaatatcca ggagaaaatt gcttttattt 3600
   tcaataatct ctcacagtca aatatgacac aaaaggttga agagctaaag gaaacggtga 3660
   aagaagaatt tatgccttgg gtttcacagt atctggttat gaagagagtc agtattgagc 3720
   caaactttca tagcctgtat tcaaacttcc ttgacacgct gaagaatcct gaatttaaca 3780
   agatggttct gaatgagacc tacagaaaca ttaaagtgct cctgacctct gataaagctg 3840
   cagccaattt ctcagatcgt tctttgctga agaacttggg acattggcta ggaatgatca 3900
   cattagctaa aaacaaaccc atcttacaca ctgacttgga tgtgaaatca ttgctgctag 3960
   aggcttatgt taaaggacaa caagaattgc tctatgtagt gccctttgtt gccaaagtct 4020
   tagaatctag cattcgtagt gtggttttta ggccaccaaa cccttggaca atggcaatta 4080
   tgaatgtatt agctgagcta catcaggagc atgacttaaa gttaaacttg aagtttgaaa 4140
   tcgaggttct ctgcaagaac cttgcattag acatcaatga gctaaaacct ggaaacctcc 4200
   taaaggataa agatcgcctg aagaatttag atgagcaact ctctgctcca aagaaagatg 4260
   tcaagcagcc agaagaactc cctcccatca caaccacaac aacttctact acaccagcta 4320
   ccaacaccac ttgtacagcc acggttccac cacagccaca gtacagctac cacgacatca 4380
   atgtctattc ccttgcgggc ttggcaccac acattactct gaatccaaca attcccttgt 4440
   ttcaggccca tccacagttg aagcagtgtg tgcgtcaggc aattgaacgg gctgtccagg 4500
   agctggtcca tcctgtggtg gatcgatcaa ttaagattgc catgactact tgtgagcaaa 4560
   tagtcaggaa ggattttgcc ctggattcgg aggaatctcg aatgcgaata gcagctcatc 4620
   acatgatgcg taacttgaca gctggaatgg ctatgattac atgcagggaa cctttgctca 4680
   tgagcatatc taccaactta aaaaacagtt ttgcctcagc ccttcgtact gcttccccac 4740
   aacaaagaga aatgatggat caggcagctg ctcaattagc tcaggacaat tgtgagttgg 4800
   cttgctgttt tattcagaag actgcagtag aaaaagcagg ccctgagatg gacaagagat 4860
   tagcaactga atttgagctg agaaaacatg ctaggcaaga aggacgcaga tactgtgatc 4920
   ctgttgtttt aacatatcaa gctgaacgga tgccagagca aatcaggctg aaagttggtg 4980
   gtgtggaccc aaagcagttg gctgtttacg aagagtttgc acgcaatgtt cctggcttct 5040
   tacctacaaa tgacttaagt cagcccacgg gatttttagc ccagcccatg aagcaagctt 5100
   gggcaacaga tgatgtagct cagatttatg ataagtgtat tacagaactg gagcaacatc 5160
   tacatgccat cccaccaact ttggccatga accctcaagc tcaggctctt cgaagtctct 5220
   tggaggttgt agttttatct cgaaactctc gggatgccat agctgctctt ggattgctcc 5280
   aaaaggctgt agagggctta ctagatgcca caagtggtgc tgatgctgac cttctgctgc 5340
   gctacaggga atgccacctc ttggtcctaa aagctctgca ggatggccgg gcatatgggt 5400
   ctccatggtg caacaaacag atcacaaggt gcctaattga atgtcgagat gaatataaat 5460
   ataatgtgga ggctgtggag ctgctaattc gcaatcattt ggttaatatg cagcagtatg 5520
   atcttcacct agcgcagtca atggagaatg gcttaaacta catggctgtg gcatttgcta 5580
   tgcagttagt aaaaatcctg ctggtggatg aaaggagtgt tgctcatgtt actgaggcag 5640
   atctgttcca caccattgaa accctcatga ggattaatgc tcattccaga ggcaatgctc 5700
   cagaaggatt gccccagctg atggaagtag tgcgatccaa ctatgaagca atgattgatc 5760
   gtgctcatgg aggcccaaac tttatgatgc attctgggat ctctcaagcc tcagagtatg 5820
   atgaccctcc aggcctgagg gagaaggcag agtatcttct gagggaatgg gtgaatctct 5880
   accattcagc agcagctggc cgcgacagta ccaaagcttt ctctgcattt gttggacaga 5940
   tgcaccagca aggaatactg aagaccgatg atctcataac aaggttcttt cgtctgtgta 6000
   ctgaaatgtg tgttgaaatc agttaccgtg ctcaggctga gcagcagcac aatcctgctg 6060
   ccaatcccac catgatccga gccaagtgct atcacaacct ggatgccttt gttcgactca 6120
   ttgcactgct cgtgaaacac tcaggggagg ccaccaacac tgtcacaaag attaatctgc 6180
   tgaacaaggt ccttggtata gtagtgggag ttctccttca ggatcatgat gttcgtcaga 6240
   gtgaatttca gcaacttccc taccatcgaa tttttatcat gcttctcttg gaactcaatg 6300
   cacctgagca tgtgttggaa accattaatt tccagacact tacagctttc tgcaatacat 6360
   tccacatctt gaggcctacc aaagctcctg gctttgtata tgcctggctt gaactgattt 6420
   cccatcggat atttattgca agaatgctgg cacatacgcc acagcagaag gggtggccta 6480
   tgtatgcaca gctactgatt gatttattca aatatttagc gcctttcctt agaaatgtgg 6540
   aactcaccaa acctatgcaa atcctctaca agggcacttt aagagtgctg ctggttcttt 6600
   tgcatgattt cccagagttc ctttgtgatt accattatgg gttctgtgat gtgatcccac 6660
   ctaattgtat ccagttaaga aatttgatcc tgagtgcctt tccaagaaac atgaggctcc 6720
   ccgacccatt cactcctaat ctaaaggtgg acatgttgag tgaaattaac attgctcccc 6780
   ggattctcac caatttcact ggagtaatgc cacctcagtt caaaaaggat ttggattcct 6840
   atcttaaaac tcgatcacca gtcactttcc tgtctgatct gcgcagcaac ctacaggtat 6900
   ccaatgaacc tgggaatcgc tacaacctcc agctcatcaa tgcactggtg ctctatgtcg 6960
   ggactcaggc cattgcgcac atccacaaca agggcagcac accttcaatg agcaccatca 7020
   ctcactcagc acacatggat atcttccaga atttggctgt ggacttggac actgagggtc 7080
   gctatctctt tttgaatgca attgcaaatc agctccggta cccaaatagc cacactcact 7140
   acttcagttg caccatgctg tacctttttg cagaggccaa tacggaagcc atccaagaac 7200
   agatcacaag agttctcttg gaacggttga ttgtaaatag gccacatcct tggggtcttc 7260
   ttattacctt cattgagctg attaaaaacc cagcgtttaa gttctggaac catgaatttg 7320
   tacactgtgc cccagaaatc gaaaagttat tccagtcggt cgcacagtgc tgcatgggac 7380
   agaagcaggc ccagcaagta atggaaggga caggtgccag ttagacgaaa ctgcatctct 7440
   gttgtacgtg tcagtctaga ggtctcactg caccgagttc ataaactgac tgaagaatcc 7500
   tttcagctct tcctgacttt cccagccctt tggtttgtgg gtatctgccc caactactgt 7560
   tgggatcagc ctcctgtctt atgtgggcac gttccaaagt ttaaatgcat ttttttgact     7620
   cttggccaaa atttagaaga tgctgtgaat atcattttga acttgtgtaa atacatgaaa     7680
   gagaaaacct ttgtctggaa cttcttggct ttgtgcaagc tgtgtccaag gcaagtacat     7740
   aaactggtac cttgtaatga agaggcagct gatgccatgc acttgtctga gggcatagct     7800
   ccatgtcttc tgacattcct ggtgtcccaa agaatagcaa aaagccagtt tgaatattat     7860
   gtaacttatt tttttaatgt ggacagggga ccttgaaaat cactaagtta ttaaaaatgt     7920
   ggatgtgcta gaattggata tgtccaggaa catgggaagg gctcactatt ggaatcccat     7980
   gagtttccat tttgtctcta cccaaacgta ttccaaagct gactgcattt gtaccatctt     8040
   atttcttttg gggattatac acctcagccg cctgagatgg gggtcagctc tttatataaa     8100
   gggaaaccag accaggccta aagcccaccc ctaccctcac ccccccaatc ctctcctgaa     8160
   acttaaaaac agtgggaata taggaaaggg aaccaaatct cattaattaa ttgttctccc     8220
   ccattacccc actgaatgaa tggccataca ggctaagctg aataatgaca aagttgaaag     8280
   gaccaataca gcccctttta taaggatttt gaatgttttg caaatgtatt ggtccctgtg     8340
   ttgtattttg tagccttttc ctgggcttca gctcccctac ttcttgtatg tgtatgcata     8400
   ctgtagctaa ccattaaagt catgacacac aaaaaaaaaa aaaaaaaaa                 8449
<210> 7
   <211> 5019
   <212> DNA
   <213> Homo sapiens
<400> 7
   gcgacctccg gcgccatttt gtagagaaac aagcggagtt aaccgaagag ggggtcgagg       60
   agagccggag tcggggaccc aggagtttcc tgtgtccagc gctgccggag ccgcctgagg     120
   tgccatgttt cagaacagag taagacccct ggtaaagaag aactgaagat attatacaga    180
   taccagatat agcctaatta caaagaaagc attaacctgc ctctgaggtg actaaagggg     240
   aataatggtg attttgcgcc gggctcggcc gcctgcttcc gccccaacca gcaatgaatc     300
   ttgactcgct ctcgctggcc ttgtctcaaa tcagctacct ggtggacaat ttaaccaaga     360
   aaaattaccg agccagccag caggaaatac agcatattgt gaatcggcac ggtcctgagg     420
   cagacaggca tttattacgc tgcctatttt cgcatgtgga tttcagtggc gatggtaaaa     480
   gcagtggcaa agatttccat cagactcagt ttctgattca ggagtgtgcg ttgctgatta     540
   caaagccaaa ttttatctcg acgctgtcct atgccattga taatccattg cactatcaga     600
   agagtttaaa gcctgcaccc cacttatttg cccagctgag taaagtgctc aaattaagca     660
   aagtacaaga ggtaattttt ggccttgccc tgttgaattc ttccagctca gatcttagag     720
   gtttcgctgc ccagtttatc aaacagaagc ttccagatct tctgcgttct tacattgacg     780
   cagacgtcag tggaaatcaa gaaggtggct tccaagatat agcaatagag gtcctacacc     840
   tcctcctctc ccatctcctc tttgggcaga agggagcctt tggagttgga caagaacaga     900
   tagacgcttt tcttaagacg ctgcgcagag attttcccca agaacgctgt cccgtggtgc     960
   tcgcaccact tttataccct gaaaaacggg acattctaat ggacaggatc ctgcctgatt    1020
   ccggaggggt agctaaaacc atgatggaga gctctttggc tgatttcatg caagaagtag    1080
   gctatggctt ttgtgcaagt attgaagaat gtcgcaatat aatcgtgcag tttggtgttc    1140
   gggaggtcac agctgcccag gttgcaaggg ttttgggaat gatggctcga actcattcag    1200
   gattaacaga tggcattcca ttacagagta tttctgctcc gggcagtggg atctggagtg    1260
   atgggaaaga taaaagtgat ggagcacagg cacacacatg gaatgtagaa gtcttgattg    1320
   acgttcttaa agaactgaat ccaagtttga atttcaagga agtaacttat gaactggacc    1380
   atcctggatt tcaaattcgt gacagtaaag gacttcataa tgtggtttat ggcattcaga    1440
   ggggtttggg tatggaagtg ttcccagtag acctcatata tagaccttgg aaacatgctg    1500
   aaggccagct ctccttcatt caacattccc ttataaatcc agagatcttc tgttttgctg    1560
   actatccctg tcatactgtt gccactgata ttctgaaagc accaccagag gatgacaatc    1620
   gagaaattgc cacatggaag agcttggatt tgattgaatc tctgctgagg cttgcagagg    1680
   ttgggcagta tgagcaagtc aaacagctct tcagcttccc tatcaaacac tgtccagaca    1740
   tgctggtatt ggccttacta caaattaaca cctcttggca taccttgcgc catgaactta    1800
   tctccactct gatgccaatt ttccttggaa accatcctaa ctcagctatt attttgcact    1860
   atgcatggca tgggcaggga cagtctccct caattcgcca acttatcatg catgcaatgg    1920
   cagaatggta catgagaggg gagcagtatg atcaggccaa attgtctcga atacttgatg    1980
   tggcccagga cttgaaggcc ttgtcaatgc tgctaaatgg tactccattt gcctttgtta    2040
   ttgaccttgc tgcacttgct tcacgtcgtg aatacctcaa acttgataag tggctcacag    2100
   ataaaattcg agagcatggg gagcctttta tccaggcgtg tatgactttt ttaaagagac    2160
   ggtgtccttc tattttgggc ggacttgccc cagaaaaaga ccagcccaaa agtgctcaac    2220
   ttcctccaga aactttggcg acaatgttgg cctgtctgca agcttgtgca gggagtgttt    2280
   ctcaggagct atcagaaact atcctcacca tggtagccaa ttgcagtaat gttatgaata    2340
   aggccagaca accaccacct ggagttatgc caaaaggacg tcctcctagt gctagcagct    2400
   tagatgccat ttctcctgtt cagattgacc ctcttgctgg aatgacatct cttagtatag    2460
   gtggttcagc tgcccctcac acccagagta tgcagggttt tcctccaaat ttgggttctg    2520
   cattcagtac ccctcagtca ccagcaaaag catttccacc cctttcaacc cccaatcaga    2580
   ccactgcatt cagtggtatt ggaggacttt catcacagct tccagtaggt ggtcttggca    2640
   caggcagcct gactggtata ggaactggtg ctcttggact ccctgcagtg aataacgacc 2700
   cttttgtaca gaggaaactg ggcacctctg gactgaatca gcctacattc cagcagagta 2760
   agatgaaacc ttcggacttg tctcaggtgt ggccagaggc aaaccagcac tttagtaaag 2820
   agatagatga tgaagcaaac agctatttcc agcgaatata taatcatcca ccacatccaa 2880
   ccatgtctgt tgatgaggta ttagaaatgc tgcagagatt taaagactct actataaaga 2940
   gggaacgaga agtatttaac tgtatgctaa ggaacttgtt tgaagaatat cgtttttttc 3000
   cccagtatcc tgataaagag ttacatataa cagcctgcct atttggtggt ataattgaga 3060
   aaggactggt cacttacatg gcactaggtc tggctctacg atatgttctt gaagccttac 3120
   gcaagccttt tggatccaaa atgtattatt tcgggattgc tgcactagat agatttaaaa 3180
   acagattgaa ggactatccc cagtattgtc agcatttggc ttctatcagt cactttatgc 3240
   aatttccaca tcatttacag gagtatattg agtatggaca gcagtctaga gatcctcctg 3300
   tgaaaatgca aggctctatc acaacccctg gaagtattgc actggctcag gcccaggctc 3360
   aggcccaggt tccagcaaaa gctcctcttg ctggtcaagt tagcactatg gtaaccacct 3420
   caacaactac cactgttgct aaaacggtta cggtcaccag gccaactgga gtcagcttta 3480
   agaaagatgt gccaccttct attaatacta caaatataga tacgttgctt gtggccacag 3540
   atcaaactga gagaattgtg gagcccccag aaaatatcca ggagaaaatt gcttttattt 3600
   tcaataatct ctcacagtca aatatgacac aaaaggttga agagctaaag gaaacggtga 3660
   aagaagaatt tatgccttgg gtttcacagt atctggttat gaagagagtc agtattgagc 3720
   caaactttca tagcctgtat tcaaacttcc ttgacacgct gaagaatcct gaatttaaca 3780
   agatggttct gaatgagacc tacagaaaca ttaaagtgct cctgacctct gataaagctg 3840
   cagccaattt ctcagatcgt tctttgctga agaacttggg acattggcta ggaatgatca 3900
   cattagctaa aaacaaaccc atcttacaca ctgacttgga tgtgaaatca ttgctgctag 3960
   aggcttatgt taaaggacaa caagaattgc tctatgtagt gccctttgtt gccaaagtct 4020
   tagaatctag cattcgtagt gtggttttta ggccaccaaa cccttggaca atggcaatta 4080
   tgaatgtatt agctgagcta catcaggagc atgacttaaa gttaaacttg aagtttgaaa 4140
   tcgaggttct ctgcaagaac cttgcattag acatcaatga gctaaaacct ggaaacctcc 4200
   taaaggataa agatcgcctg aagaatttag atgagcaact ctctgctcca aagaaagatg 4260
   tcaagcagcc agaagaactc cctcccatca caaccacaac aacttctact acaccagcta 4320
   ccaacaccac ttgtacagcc acggttccac cacagccaca gtacagctac cacgacatca 4380
   atgtctattc ccttgcgggc ttggcaccac acattactct gaatccaaca attcccttgt 4440
   ttcaggccca tccacagttg aagcagtgtg tgcgtcaggc aattgaacgg gctgtccagg 4500
   agctggtcca tcctgtggtg gatcgatcaa ttaagattgc catgactact tgtgagcaaa 4560
   tagtcaggaa ggattttgcc ctggattcgg aggaatctcg aatgcgaata gcagctcatc    4620
   acatgatgcg taacttgaca gctggaatgg ctatgattac atgcagggaa cctttgctca    4680
   tgagcatatc taccaactta aaaaacagtt ttgcctcagc ccttcgtgta agttggctat    4740
   ttccttggta taggtacaaa acgtattact gcttgtctgt aataattttt ttctttgtct    4800
   atatatggca ctgggcgtta ccacttattc ttaataatca ccatatttgt ttgatgtctt    4860
   ccatcatttt agattgtaat tctgtgaggc aaagcatcat gtctgtgtgt tttttttttt    4920
   ttctgttata ttctcaacac gatgtttgac agatagtaga tacccaaata tttgttggtt    4980
   taaataaatt aagatttaaa aaggaaaaaa aaaaaaaaa                           5019
<210> 8
   <211> 4936
   <212> DNA
   <213> Homo sapiens
<400> 8
   gagccaggct tggcggtcct cagaggggct gataaatcgg cgctcgactg gcctaggagc      60
   tgtccgcagg gagatttatg acaaagtcca gttttcaaca atgctgaaat caaatgattg     120
   cctgttttct ttggaaaatt tgttttttga aaaaccagat gaagttgaaa accatccaga     180
   caatgaaaag tcattggatt ggtttctccc tcctgctcca ttgatttcag aaattccaga     240
   tactcaggag ttagaggaag aattagaaag tcataaactg ttaggtcagg aaaagaggcc     300
   aaaaatgtta acatcaaatt taaagataac taatgaagat acaaattata tttcactaac     360
   acaaaaattc cagtttgcct ttccttctga taaatatgaa caggatgatc taaatttaga     420
   aggggtaggt aataatgact tatcacatat tgctggcaag ctgacatatg cttctcagaa     480
   atataaaaat cacattggca ctgagatagc acctgagaag agtgttcctg atgatacaaa     540
   attagttaat tttgcagaag ataaaggaga gagcacatca gtattccgga aaagattatt     600
   taaaatatct gacaatatac atgggagtgc ttattctaat gacaatgaat tggactctca     660
   cattggctca gtgaaaattg tacaaacaga aatgaacaaa gggaaatcaa ggaactatag     720
   caatagtaag caaaaatttc agtattctgc aaatgtgttt acagcaaata atgctttttc     780
   tgcttctgaa atcggagaag gcatgttcaa agcaccatct ttttcagttg ctttccaacc     840
   tcatgatatt caagaggtaa cagaaaatgg tttaggttcc ttgaaggctg tcacagaaat     900
   tccggcaaaa tttagaagta ttttcaaaga atttccatat ttcaactata tacagtccaa     960
   ggcctttgat gatcttcttt acacagatag gaattttgtg atttgtgctc caactggttc    1020
   tggaaaaact gtagtgtttg aactagctat aacaagattg ttaatggaag taccattgcc    1080
   atggttgaat attaaaattg tttacatggc accaataaaa gccttgtgca gtcagcgttt    1140
   tgatgactgg aaagaaaaat ttggaccaat aggattgaat tgtaaagaac ttactggaga    1200
   tacagtaatg gatgatctat ttgagattca gcatgcccat attattatga caactccaga 1260
   aaaatgggat agcatgacta ggaaatggag agacaactct ttggttcagc tggttcgact 1320
   gtttctcatt gatgaggtac atattgtaaa agatgaaaat cgtggtccaa ctcttgaagt 1380
   tgtagttagc agaatgaaaa ctgtacagtc tgtttctcag actttaaaaa ataccagcac 1440
   tgctattcca atgcgatttg tagctgtatc tgcaacaatt ccaaatgctg aggatattgc 1500
   agaatggctt tcagatggtg aaagaccagc tgtgtgtctg aaaatggatg agagccatag 1560
   accagtgaaa cttcagaaag tggtccttgg atttccctgc agtagtaacc aaactgagtt 1620
   taagtttgat ttaaccctca actacaaaat tgccagtgtt atacaaatgt actctgatca 1680
   gaaacccaca cttgtgtttt gtgcaacaag gaagggtgtg caacaggctg cttctgttct 1740
   tgtgaaagat gctaaattta ttatgactgt ggaacagaaa cagaggttac agaagtatgc 1800
   atattccgta agagattcaa aactgagaga tatcttaaaa gatggtgctg cttatcatca 1860
   tgctggtatg gagctgtcag atagaaaagt agttgaggga gcttttactg ttggagattt 1920
   accagttctt tttactacca gtactttagc tatgggagta aatttgcctg ctcacctagt 1980
   agttataaaa tctacaatgc attatgctgg aggactgttt gaagagtaca gtgaaacaga 2040
   tattctacag atgattggta gagctggtcg acctcaattt gacactacag ctactgcagt 2100
   tatcatgact cgattaagca caagggacaa gtacattcag atgttagctt gtagagacac 2160
   tgtagaaagc agtttgcaca gacatcttat tgaacattta aatgcagaga tagtactgca 2220
   taccatcacg gatgtgaata ttgctgtgga atggatacga tcaactctgc tttatatcag 2280
   agccttgaaa aatccatctc attatggttt tgcatctgga ttgaacaaag atggaattga 2340
   agcaaaatta caagaattat gtttgaagaa tctgaatgat ttatcatccc tggacttaat 2400
   aaagatggat gaaggtgtta atttcaaacc aactgaagca ggaagattga tggcttggta 2460
   ttatattaca tttgagacag tgaagaaatt ttatacaatc agtggaaaag aaaccttatc 2520
   agatctggtt acattgatag ctggctgcaa ggaatttcta gatatacagt taaggataaa 2580
   tgaaaagaaa acactgaata ctttgaacaa agatccaaat cggataacta tcagatttcc 2640
   aatggaagga agaattaaaa caagagaaat gaaagtaaat tgtcttattc aggctcaact 2700
   aggatgcatt cccatacaag attttgcttt gacacaagat accgcaaaga ttttcagaca 2760
   tggctcccga attacaagat ggttgtcaga ttttgtagct gctcaagaaa agaagtttgc 2820
   tgtactattg aatagtttga ttttagctaa atgttttagg tgtaaacttt gggaaaactc 2880
   tctgcatgta tccaaacaac tggaaaaaat tggtataaca ttgtcaaatg caatcgtaaa 2940
   tgctggtttg acttccttta aaaaaataga agagacagat gcaagggaac ttgaattgat 3000
   tttaaacaga catcccccct ttggaaccca gataaaagaa actgtgatgt atctaccaaa 3060
   atatgaactt aaagtggaac agattacaag atatagtgat acgacggcag aaatattagt 3120
   gactgttata ttaagaaatt ttgaacagct acaaactaaa agaacagcat cggattctca 3180
   ctatgttacc ttaatcatag gtgacgcaga taatcaagta gtttatctgc acaagattac 3240
   ggattctgtt ttgctaaaag ctggaagttg ggctaaaaag attgctgtga aaagagctct 3300
   taaatctgaa gatcttagca taaatctaat aagttctgaa tttgttgggc ttgatattca 3360
   gcagaaactt acagtctttt acttagaacc caagaggttt ggaaatcaaa tcactatgca 3420
   aagaaaatct gaaacacaga tttcccattc taaacattca gacatatcta caatagcagg 3480
   acctaataaa ggaacgactg ccagcaaaaa acctgggaac cgagaatgca atcatctttg 3540
   taaaagtaaa catacatgtg gacatgactg ctgtaaaatt ggagttgcac agaagtcaga 3600
   aattaaagag tcaacaattt cttcatattt atctgattta agaaacagga atgctgtttc 3660
   atctgttcct ccagttaaac gtctgaagat acagatgaat aaatctcaaa gtgtggacct 3720
   taaagagttt ggttttactc caaaaccttc ccttcctagt atatcaaggt cagaatattt 3780
   aaacatatct gaattgccta taatggagca gtgggatcag cctgaaatct atggaaaagt 3840
   tagacaagaa ccatctgaat atcaagacaa agaagttttg aatgtgaact ttgaattggg 3900
   aaatgaagtt tgggatgatt ttgatgatga aaacttagaa gttactagct tttcaactga 3960
   tactgagaag acaaaaatat caggatttgg aaacactttg agttcaagta ccaggggaag 4020
   taagctaccc cttcaagagt caaagagcaa attccaaaga gaaatgtcaa acagttttgt 4080
   ttcatcacat gagatgtcgg atatttcttt atcaaattct gctatgccca agttcagtgc 4140
   atcctccatg acaaaattac ctcaacaagc cggaaatgca gttattgtcc attttcaaga 4200
   aagaaaacca caaaatctgt caccagagat tgagaagcaa tgctttactt tctctgaaaa 4260
   aaacccaaat tcttcaaatt ataaaaaagt ggattttttt attagaaaca gtgaatgtaa 4320
   aaaggaagtt gatttcagta tgtatcatcc tgatgatgaa gctgatgaaa tgaagtcttt 4380
   attgggaata tttgatggta ttttctaaaa caaacaaata ctttttatat tgataagaga 4440
   aagaataaag acacctaatc acaaagcatg ctttttattt ttaaaaagta gttcataatt 4500
   gttcggattt ttttctcctg tatttgatta gctttgttca ctttttgcaa caaaatgtat 4560
   gggccgattt attttcccat tatattgtaa cttcttgaaa agctctttta cagtggcttt 4620
   gtttcaataa gcccttaagt taagggctga ttgcacttag aaataagttg cacttagaaa 4680
   taattatttg gctattaatt ggattttgac aagaaccaat gtaaaatttc atttttatat 4740
   tttcttaaca agaagaaaaa tattctatga aagcaaagta atgctaactt aatgaatctg 4800
   cttattgtat ggattaatca ctaatattat tcccatgtat tttgttttac taaagatttt 4860
   taagaatcca gtggctattt gaacctactg ttatctaact ttgagcaaca ataaatgaaa 4920
   cattaatcag taaaaa 4936
<210> 9
   <211> 2272
   <212> DNA
   <213> Homo sapiens
<400> 9
<210> 10
   <211> 4851
   <212> DNA
   <213> Homo sapiens
<400> 10
   ggcggaggga gctggagctg tcacagtccc cgggtgcgcc tgaccgagcc aagtggggtg 60
   tcatggccgc ggggggcagc ggctgcactt cctcagcggg cggcggcggc cggggagtta 120
   atcctcgacg cacgggtcgg tgtgtcccgt ttccctctgt gcggcggccg gcgggaccat 180
   aagggcttaa ctcatatatt taacccccct ccaaaaaggt ttgaaagtat tcttgaaggg 240
   ctgtttggac ctgcattatt aaaagatctc agtttattta aagactgtga acctgaaagc 300
   atttctgatt ggacttttga tgaaaactgt ttattctgtt gcttgagaag agataaagta 360
   aagggacact tagtcggtct ggatgaacca gcttcgggag ctgggcaaga agctctgctt 420
   aaacaagagc aagcaaaaat cattcgattc gagagacaag cagaagaatt cctcaatgca 480
   gtcttttata gaaaagacag tccctgggtc tccgacccca atattcccct agtggcccgt 540
   gagatcatgc agcgaatgat ccaacaattt gctgctgaat atacctcaaa aaatagctct 600
   actcaggacc ccagccagcc caatagcaca aagaaccaaa gcctgccgaa agcatctcca 660
   gtcaccacct ctcccacggc tgcaactact cagaaccctg tgctcagcaa acttctcatg 720
   gctgaccaag actcacctct ggaccttact gtcagaaagt ctcagtcaga acctagcgaa 780
   caagacggtg tacttgatct gtccactaag aaaagtccat gtgctggcag cacttccctg 840
   agccactctc caggctgctc cagtactcaa gggaacgggc gacctgggag acccagccag 900
   taccgcccag acggacttcg gagtggtgat ggggtacctc caagaagctt acaggatgga 960
   accagggaag gttttggaca ctccacatca ctcaaagttc cactggctcg atccctgcag 1020
   attagtgaag aactactgag cagaaaccaa ttgtccacag ctgccagcct tgggccatct 1080
   ggattacaga atcatggaca acacttaata ttatccaggg aagcctcttg ggcaaaacca 1140
   cattacgagt tcaacctcag ccgtatgaag ttcaggggaa atggtgcact cagcaacatc 1200
   agtgaccttc cttttcttgc agaaaactct gcctttccaa aaatggcact tcaagcaaaa 1260
   caagatggaa aaaaggacgt gagccattca tctcctgtag atttaaagat accacaagtt 1320
   cgaggaatgg atctttcttg ggagtctcgc actggtgatc agtacagcta tagctctttg 1380
   gtaatgggtt cacaaacgga gagcgcgctt agtaaaaaat taagggctat tcttccaaaa 1440
   caaagtagaa aaagcatgtt agatgctggg cccgattctt ggggctcaga tgctgagcag 1500
   tctacccctg gacagccata tcccacatcg gatcaagaag gagaccctgg ctccaagcag 1560
   cctcggaaga aaagagggcg ttacagacag tacaacagtg agatactgga ggaagcaatc 1620
   tcagtggtta tgagtggaaa aatgagtgtt tccaaagctc agagtattta tgggattccc 1680
   cacagtacac tggagtacaa agtaaaggag aggctgggca ctttgaaaaa ccctccaaag 1740
   aaaaagatga aattaatgag gtcggagggg ccagatgttt ctgtaaagat tgaattagat 1800
   ccccagggag aggcagcaca aagtgcaaat gaatcaaaaa acgagtagga atactgtaga 1860
   gtgccaatta ctgtacaaac tgggtgagca ctactgcatc attgttcagc tatcattgct 1920
   tgcacgtaat ttcatttact gtgacacttg cttctttgca gattttgcat tgacttgtgt 1980
   gtacagagat gaaatgtgca ttctgaatgt tgcatatttt aaatttttca tgtgcagtat 2040
   ggctcggaat atgtttggcc ttttgcatgt ttctctacaa aagagaattg agttacctca 2100
   cagagaacag atacatggaa gtggactcct tgcctgtaga gcctgcatgc ttttttgttt 2160
   ttttttgttg ttgttgtttt ttcttaagtt atatttgttt ttacttttta aaaaagaaga 2220
   ggaaacaaag ccccctttta gaaactacgt ctgtcatact gggagcttta tcactgcaaa 2280
   ttggaaagcc atcttataca aaattattca catttttcat ctacgattca actaatcgaa 2340
   ggattaaact aaagaaaaaa ctaagaagaa caatgaacct ttgactttga gaaatttggt 2400
   tattcactga cataattatt gggtcattta tagttacatt ttataatttt cttctcacta 2460
   ttaaatttat ttaaattagc aagctattag tgctcaccca gaggggaagg tggtggaaaa 2520
   tgtgcacaca ctaccttcag aaatgcttca gttaattact ttgaacacta cctttgctgt 2580
   aatttcattt gagattttct aagggtagaa tttggtctca ccaacaagtg aggatatagc 2640
   cttatctcat ggaggacgag ctccgtattt actcaggagc agtcagggta cattacataa 2700
   aacaatggag gatgcctcat tttagcaaac taggtttctt tgtattcttc agtcctttta 2760
   cagaattgat gtgctaactg aatatcattg cagcaactag actaagatat tcaagatctc 2820
   tttattgggg atgggagaaa tagggaaaga aatgtgtata agtaattatg atattgcaaa 2880
   agtgatactt agattttaca gcctcagtag tctgcccagt gtccacatta atgaaggatc 2940
   catgtttgta gtgagagaaa aaaaccccaa ggtaacccga tatgatttag gatgcatatc    3000
   agttctaaca attcaatcag aagtcaagct cattggaatt ccttttttaa ctgatccaaa    3060
   tactagtaga agggggaggg agaggtgttg ggtttttttt ttaagttttt atttaatttt    3120
   gttggttaga atttttttct gtttttggca tcctacataa tacccccctt cttgactttt    3180
   tctgataatt agctgatatt catggttgtt tagcacacag ttcaggacct ttgagatcat    3240
   gtttgtataa gcactccttg aagaatatct aagctttttc tgagatgggc ttttgaaatt    3300
   ataataaggg aagtttattt tctgcggttt ttgcaagaat aagcaaagcc catggaattt    3360
   aatttttcat tgtgatcaga ttaaaattac tatttgtgca aaacaaattc cacaccacat    3420
   gtactgtata ttctaatctg ctgagaaagg tggtcaggcc cttctaaatg cttaaccaaa    3480
   aacaaacatt attggagttt cagtgtaaaa taaaattaaa attggagggt tggttagatt    3540
   gatttcagat ggatactaac agtttgaggg gaggtccaga tatttccaag gcataaattc    3600
   ttgcctggaa actttggagc aattaattaa attaggttaa gttttccctc agcaacctgt    3660
   gtgttcaaag gttaggcaca ccattgctgt tactaaaata catgcactgc tcattcttga    3720
   catagcttgg gtgtgggttc ctcttgtttt ggttattttg tgtagcagtt taatcagtgg    3780
   ataactgagc ctgtttcctc ttagctttct agacttctta acagaaaaaa cagtttgggt    3840
   tcaatagcat tttatgattc tacaatattt tgtcacctag ctggaagttg caataaaaat    3900
   tccttatgaa atatatggtt ttcatctgtg attgaaggaa atcatagaaa gtttgctgaa    3960
   agcaaagggc taagcttatg aaggaatgcc tcctttgtta cacactcaag aatttgtcag    4020
   cacacaatta attgcagcca tagtaccttt ttgcgtaacg tttgggatat tcagtagccc    4080
   aggattgcta caaaaaggtc ctttgagaaa attaatttgt gcttgctttt atcccctctc    4140
   agaaagacat cactaaaggc ccattaaaaa tctggcccta aaagttcaac ttttgtagag    4200
   tttattgttc agagaggttt aatcctatgt ggtagtctat ataagtgatg tatgagttga    4260
   gttatgtaaa ttttgtcatt gtagtgtaca tggagtgatc attttactaa cataaatatt    4320
   ttctatgtgt gtttcagtgg atgacaatcg agcagcagaa gaatggtgtg cctacccttt    4380
   aatgctgcag tttaagagaa cttgtattgt gtcatttcag attgtaggct gagagttgta    4440
   aatagtgaaa atttgagtac ttctattatt tgttttggtt agaaagtgaa tttaaaaaac    4500
   aaaccaaact ctaaactttc tcagattaaa agtcctgaga cctgaagatt gtaatattca    4560
   tgtctgtgaa gcttttaaac attacacttg agatcagtca tgacttgata ttcaggtaaa    4620
   ttttcttttc caagaagctt ttgaataagc atatttcctc caaaggtctc tctctttctc    4680
   tctctttttt tgagtgtaga ttattttaaa gcactaattg cattacattg gtaactgcaa    4740
   tataaaatag ccattattgt tttgtgaagc atggttgaaa ttagctagtg gtcccttttg    4800
   aatttcatga gcctctcaaa aaagaaaaac ttaagaaaaa aaaaaaaaaa a             4851
<210> 11
   <211> 1107
   <212> DNA
   <213> Homo sapiens
<400> 11
   atggaacctg ggcagccccg ggagccccag gagccccgcg agcccgggcc aggagcggag      60
   accgctgcgg ccccggtctg ggaggaagcc aagattttct acgacaacct cgcgcccaag     120
   aagaaaccca aatcgcccaa gcctcagaat gcagtcacca tcgctgtgtc ctcccgagcc     180
   ttgtttcgca tggacgagga gcagcagatc tacacggagc agggcgtgga ggagtacgtg     240
   cgctaccagc tggaacatga gaacgaaccc ttcagtcccg ggccagcctt cccttttgtg     300
   aaggctctgg aggccgtgaa caggcggctg cgggagctgt accctgatag tgaggacgtc     360
   ttcgacatcg tcctcatgac taacaaccat gctcaagtgg gtgtccgcct catcaacagt     420
   atcaaccact atgacctgtt catcgagagg ttctgcatga caggtgggaa cagcccgatc     480
   tgctacctca aggcctatca caccaacctc tacttgtcag ccgatgcgga aaaagtgcga     540
   gaagccattg atgaggggat cgcagctgcc accatcttca gccccagcag ggatgtggtt     600
   gtgtcccaga gtcagctgcg cgtggccttc gatggggacg ccgtgctctt ctcggacgag     660
   tcggagcgca tcgtcaaggc ccacgggctg gaccgattct tcgagcatga gaaggcccac     720
   gagaacaagc ctctggctca gggcccctta aagggctttc tggaggcact gggtaggttg     780
   cagaagaagt tctactccaa aggcctgcgg ctggagtgcc caattcgtac ctacttggtg     840
   acagcacgca gtgcagccag ttccggggcc cgggctctca agaccctgcg cagctggggc     900
   ctggagacag atgaagcctt gttccttgct ggagcgccca agggccctct ccttgagaag     960
   atccgcccac acatcttctt tgatgaccag atgttccatg tggctggggc tcaggagatg    1020
   ggcactgtgg ccgcccatgt gccttatggt gtggcacaga caccccggcg gactgcacct    1080
   gcaaagcagg ccccatctgc acagtag                                        1107
<210> 12
   <211> 3000
   <212> DNA
   <213> Homo sapiens
<400> 12
   caggccacac cggtggtctg ggctggggac cgcgggtcgg gtccggtttc cagggggttc      60
   ctggtgctcg aggctggcgg cgaggaagga ccagatggcc tttgaggatg tggctgtgta     120
   cttctcccag gaggagtggg ggctcctgga cacagcccag agggccctgt accgccgcgt     180
   gatgctagac aacttcgcac ttgtggcctc gctgggactc tccacctctc gacctcgtgt     240
   ggtcatccaa ctggagcgtg gcgaggagcc ctgggttccc agtggaacgg acacaaccct     300
   gtccaggacc acctacagga ggcgcaaccc tggttcctgg agtttgacag aggatagaga 360
   tgtttctgga gaatggccac gagctttccc agatacccca cctgggatga ctactagcgt 420
   cttccctgtt gccggtgcct gccacagtgt aaaaagcctg cagagacaac ggggtgcctc 480
   cccatctcgg gagagaaaac ccacgggggt gtcggtgatc tactgggaga ggctcctgct 540
   aggctcaggc agtgggcaag ccagcgtcag cctgcgactg acctccccgc ttaggcctcc 600
   cgagggcgtc cggcttaggg aaaagacact cacagagcat gcgttgctgg ggaggcagcc 660
   caggacgcct gagcggcaga aaccatgtgc acaggaggtc cctgggagaa cctttgggag 720
   cgcccaggac ctggaggctg ccggcggtcg gggacatcac cgaatgggtg cagtttggca 780
   ggagcctcat agactcctcg gtggccagga gccctcgacc tgggacgagc tgggcgaggc 840
   tcttcacgct ggggagaagt ccttcgaatg cagggcgtgc agcaaagtgt tcgtgaagag 900
   ctccgacctc ctcaagcacc tacgcaccca caccggggag cggccctacg agtgcgccca 960
   gtgcggcaag gccttcagcc agacgtcgca cttgacgcag caccagcgca tccacagcgg 1020
   cgagacgccc tacgcgtgcc ccgtgtgcgg caaggccttc cggcatagct cctcgctggt 1080
   gcggcaccag cgcatccaca cggccgagaa gtccttccgc tgctccgagt gcggcaaggc 1140
   cttcagccac ggctccaacc tcagccagca ccgcaagatc cacgcgggtg ggcgtcctta 1200
   tgcttgcgca cagtgtggcc gccgcttctg ccgcaactcg cacctgatcc agcacgagcg 1260
   tacgcacaca ggcgagaagc ccttcgtgtg cgcgctctgc ggtgctgcct tcagccaggg 1320
   ctcctcgctc tttaagcacc agcgcgtgca cacaggcgag aagcccttcg cctgcccaca 1380
   gtgcggccgc gcctttagcc acagctccaa cctcacccag caccagctcc tgcacacggg 1440
   cgagcggccc ttccgctgcg tggactgtgg caaggccttc gccaagggcg ccgtgctgct 1500
   cagccaccgg cgcattcaca cgggcgagaa gcccttcgtg tgtacgcagt gtggccgcgc 1560
   cttccgtgag cgcccggccc tcttccacca ccagaggatc cataccggcg agaagaccgt 1620
   ccggcgatcc agggccagcc tgcaccccca ggccaggtct gttgccgggg catcatcaga 1680
   aggtgcgcca gcgaaggaaa ccgagcccac tcccgcctcg ggcccagccg ccgtctcgca 1740
   gccagcggag gtctgaggtc acaggttgca gccctggcct tctgtgaatc ccttccacag 1800
   ctaaagggca tatgtcctct gcagatccac agcagagaaa aagtcccgtg cttgctagtc 1860
   agggacaagg gaggcccttt ggctgtgatt tcatttgcac gtggggacag gatttgccag 1920
   ttcacccaca gatcacacct ccatccccaa agaggtagca ctgcagcaac atcaggggga 1980
   ggacgtggtg gctgaactct agtggggccg agactattca gagccagtag gaggccgaca 2040
   gtcacagcac tgcactgtgg tgcggcttca tgtgatatga cagtggatgc taaggtgaga 2100
   gggatgcagg catgggttgg gggtggccca gagaaactta tgacagctgt acacaaactg 2160
   gccgctggag agatgcccgc tgagggtatt ctcccctcaa cccactgcct ctgttcatcc 2220
   aagacttcct aggggccagc ctagcagaca agagaccaca agggactggg gatcagggtc 2280
   tgggctctgt cagccgccac ctctgggaaa gagaaaaggt ttgggtccac tgaacatcat 2340
   gtttgtagac gctgacaggt ggggtcctaa tgagagccaa cacatgctca ctgccagctc 2400
   ctgtcctgag tactgggaag tttctcctga agccctgtga gatggctctg tggctggtat 2460
   cccgacttgg aagatgagga aactgaggca cacggcctgg cctggcttca cacacatagc 2520
   cgactcagga gagggatgcc catgggggaa catgtgactc tcagcattgg aaggacagag 2580
   ctaggatgat ggctttccgg tggcactcgt tcaggttttt gcccaagtct cagcttggcc 2640
   aaggcctgtc actgactggt ttaccaaagt cgatgtgagg aggaggcttt atacctgagg 2700
   ggatgatgtt aacttcagac aagatggagc tgctcacttt tgccgggttt ggtggccact 2760
   tcacccccaa ccctgtctca cccccattat ccctcctcaa ttggaggctg gacagagctg 2820
   aataggaaag acttgctatt gcctaaggct atgtgtgaca ccctcctgag gacctcccca 2880
   ccccagtgta atggcccttc atggcaggga cagaaaggtg gactgggggc catttgcttc 2940
   ctgtggcctt cagcagacca ggccctgtcc ctacctggag cctcacctcc aaggaaattc 3000
<210> 13
   <211> 2001
   <212> DNA
   <213> Homo sapiens
<400> 13
   cggccttcct gtacagcgac ggccagtccg agaccatcct tggcggcctg gggctccgaa 60
   tgggcagcag cgactgcaga gtgaaaattg ctaccaaggc caatccatgg attgggaact 120
   ccctgaagcc tgacagtgtc cgatcccagc tggagacgtc actgaagcgg ctgcagtgtc 180
   cctgagtgga cctcttctat ctacatgcac ctgaccacag cgccccggtg gaagagacac 240
   tgcgtgcctg ccaccagctg caccaggagg gcaagttcgt ggagcttggc ctctccaact 300
   atgccgcctg ggaagtggcc gagatctgta ccctctgcaa gagcaacggc tggatcctgc 360
   ccactgtgta ccagcttctg gaaggagcac cacttcgagg gcattgccct ggtggagaag 420
   gccctgcagg ccgcgtatgg cgccagcgct cccagcatga cctcggccgc cctccggtgg 480
   atgtaccacc actcacagct gcagggtgcc cacggggacg cggtcatcct gggcatgtcc 540
   agcctggagc agctggagca gaacttggca gcggcagagg aagggcccct ggagccggct 600
   gtcgtggacg cctttaatca agcctggcat ttgtttgccc acgaatgtcc caactacttc 660
   atctaagctc attgtggctc aggctgccca aggcttttct gtcaactctt ttgctctctc 720
   ccgctttgtc taatttagaa ctgcctcact aaattcttag ggatggaagt atttggaaaa 780
   aaacctaaca gtagagtcac cacctaagga agaataaaat ctcccagggt gctgtgtgtt 840
   agtctgtttg cgttgctata aaagaatacc tgagactggg tcatgtataa agaaaagagg 900
   tttctttggc tcacagttct gcagtctgta caagagggtg tggtgccggc atctgctctt 960
   ggtgagggcc tcaggaagct tagaatcatg gcagaagggg aaacggagcc agcgtgtcac 1020
   atggtgagag agggagcaag agacagacag gggaggaaat tcacacactg atggtgggga 1080
   tgtaaaatgg tacaaccagt ttggaaaaca gtttggcagt ttctcaaagg attaaacata 1140
   aaattaccat aggattcagc agttccactt gtgggtatgt agccaagaga aatgaaaaca 1200
   tatctcccca caaaaaaact tgggtatgag atttcacatt atcattgctc ataatagcca 1260
   ataagtaaaa acaacccaaa tgtccaagaa tgaataaatg gataaacaaa atatggtata 1320
   tttatacaac agactattat tcggccatta aaaaaaaaaa gagtggctga cacctgtaat 1380
   ctcagcactt tgagaggcca aggcagtagg actgattgaa gacaggagtt ccagaccagt 1440
   ctgggaaaca aagcgagacc ctgtctccac taaacataaa aacaaaatta ctggggcccc 1500
   atggcacaca cctgtagtcc cagctgctcg ggaagctgag atgggcggat tgcttgagcc 1560
   caggtattca agtctggagt gagctatgac tgtgccactg cactccagcc tgggcgacag 1620
   agcaaaccct gtttccaaac aaacaaacaa acaaacaaat acacagacag aagtagttaa 1680
   acatctacaa ggtaagtgca tcttgaagac gtgttaagtt aaagaagcca attacaaaag 1740
   gtttcacgtt gtatgatttc gtttatatga aatgtccaga ataggcaaat ctgtttgaga 1800
   gagagaaagt agatgagtac ttgcctagga ctgggaggag gattgcgagg aaatggagat 1860
   tcactgctaa tgagtacagg gtttcttttg ggggcgctta tgaagatgct ctgaaattga 1920
   ttgtgatggt tgtacaactc tgaatacatg aaacagcatt aaacatcact ttaagtaagt 1980
   caaaaaaaaa aaaaaaaaaa a 2001
<210> 14
   <211> 2254
   <212> DNA
   <213> Homo sapiens
<400> 14
   aaagaaccgc acaccacaga ctccctccag ctctttgtgt gtggctctct cagggtccaa 60
   caagagcaag ctgtgggtct gtgagtgttt atgtgtgctt ttattcactt cacacttatt 120
   gaaaagtgtg tatgtgagag ggtggggtgt gtgtgtcaaa gagagtgagg aagagaagga 180
   gagagagatc aattgattct gcagcctcag ctccagcatc cctcagttgg gagcttccaa 240
   agccgggtga tcacttgggg tgcatagctc ggagatgcag tccccctgga aaatccttac 300
   ggtggcgcct ctattcttgc tcctgtctct tcagtcctcg gcctctccag ccaacgatga 360
   ccagtccagg cccagcctct cgaatgggca cacctgtgta gggtgtgtgc tggtggtgtc 420
   tgtaatagaa cagcttgctc aagttcacaa ctcgacggtc caggcctcga tggagagact 480
   gtgcagctac ctgcctgaaa aactgttctt gaaaaccacc tgctatttag tcattgacaa 540
   gtttggatca gacatcataa aactgcttag cgcagatatg aatgctgatg tggtatgtca 600
   cactctggag ttttgtaaac agaacactgg ccaaccattg tgtcatctct accctcttcc 660
   caaggagaca tggaaattta cactacagaa ggcaagacaa attgtcaaga agtccccgat 720
   tctgaaatat tctagaagtg gttctgacat ttgttcactc ccggttttgg ccaagatctg 780
   ccagaaaatt aaattagcta tggaacagtc tgtgccattc aaagatgtgg attcagacaa 840
   atacagcgtt ttcccaacac tgcggggcta tcactggcgg gggagagact gtaatgacag 900
   cgacgagtca gtgtacccag gtagaaggcc gaacaactgg gatgtccatc aggattcaaa 960
   ctgtaatggc atttggggtg tcgatccaaa agatggagtt ccatatgaga agaaattctg 1020
   tgaaggttca cagcccaggg gaatcatttt gctgggagac tcagctgggg ctcattttca 1080
   catctctcct gaatggatca cagcgtcgca gatgtctttg aactctttca tcaatctacc 1140
   aacagccctt accaacgagc ttgactggcc ccaactctct ggtgctacag gatttctgga 1200
   ctccactgtt ggaattaaag aaaaatctat ttaccttcgc ttatggaaaa gaaaccactg 1260
   taatcacagg gactaccaga atatttcaag aaatggtgca tcttcccgaa acctgaagaa 1320
   atttatagaa agcttgtcta gaaacaaggt gttggactat cccgccatcg ttatatatgc 1380
   catgattgga aatgatgtct gcagtgggaa gagtgaccca gtcccagcca tgaccactcc 1440
   tgagaaactc tactccaacg tcatgcagac tctgaagcat ctaaattccc acctgcccaa 1500
   tggcagccat gttattttgt atggcttacc agatggaacc tttctctggg ataatttgca 1560
   caacagatat catcctctcg gccagctaaa taaagacatg acctatgcgc agttgtactc 1620
   cttcctgaac tgcctccagg tcagcccctg ccacggctgg atgtcttcca acaagacgtt 1680
   gcggactctc acttcagaga gagcagagca actctccaac acactgaaaa aaattgcagc 1740
   cagtgagaaa tttacaaact tcaatctttt ctacatggat tttgccttcc atgaaatcat 1800
   acaggagtgg cagaagagag gcggacagcc ctggcagctc atcgagcccg tggatggatt 1860
   ccaccccaac gaggtggctt tgctgttgtt ggcggatcat ttctggaaaa aggtgcagct 1920
   ccagtggccc caaatcctgg gaaaggagaa tccgttcaac ccccagatta aacaggtgtt 1980
   tggagaccaa ggcgggcact gagcctctca ggagcatgca cccctgggga gcacagggag 2040
   gcagaggctt gggtaaactc attccacaaa ccctatgggg gctgccacgt cacaggccca 2100
   aaggactctt cttcagcagc atctttgcaa aatgtctttc tctcaatgaa gagcatatct 2160
   ggacgactgt gcaatgctgt gtgctcccgg ggatcagtaa cccttccgct gttcctgaaa 2220
   taacctttca taaagtgctt tgggtgccat tcca 2254
<210> 15
   <211> 3689
   <212> DNA
   <213> Homo sapiens
<400> 15
   agttaaaacc aacctctcct gccctgagtg gataggtagg gttagggttg ccagatgtca 60
   cgaagttaca ggatgctcag ttttaaggta tatcccttat actataaggg ttatagtaaa 120
   aaatattcat tatgtgaaat tcaaatataa ctgggtatca ggtattctat gtggcaaccc 180
   taggtagggg agcacaggtt aggcaagcga ttagaagatt tgcagcctcc aaagtttctg 240
   cacctcgatg ggacactaga acaggaaggc tcctgggcct ttctggctct gggaatgaag 300
   cgtggaaaac cctccttagg cgggcgcagt gcttcaagta gccaagctct gacttccgag 360
   ggaagaaagg aggccatggg cctctgccag agccatgctc tgcactctgg ggtcagcaga 420
   gttcaaaacg acctgcaacg tctggcgctt agctcctaaa gaggtctcca gtccagcgcc 480
   gacggccagc ggctagaggc cgtccgcccg actccaagat ggcgcccgcc acagctgcca 540
   ggtgttaaga tggcggcgcg gggccgcgcc cgcgctccca ggctctcctc ccccagcctt 600
   cctccggctg gcagcacgac tcgcgtagcc gtgcgccgat tgcctctcgg cctgggcaat 660
   ggtcccggct gccggtcgac gaccgccccg cgtcatgcgg ctcctcggct ggtggcaagt 720
   attgctgtgg gtgctgggac ttcccgtccg cggcgtggag gttgcagagg aaagtggtcg 780
   cttatggtca gaggagcagc ctgctcaccc tctccaggtg ggggctgtgt acctgggtga 840
   ggaggagctc ctgcatgacc cgatgggcca ggacagggca gcagaagagg ccaatgcggt 900
   gctggggctg gacacccaag gcgatcacat ggtgatgctg tctgtgattc ctggggaagc 960
   tgaggacaaa gtgagttcag agcctagcgg cgtcacctgt ggtgctggag gagcggagga 1020
   ctcaaggtgc aacgtccgag agagcctttt ctctctggat ggcgctggag cacacttccc 1080
   tgacagagaa gaggagtatt acacagagcc agaagtggcg gaatctgacg cagccccgac 1140
   agaggactcc aataacactg aaagtctgaa atccccaaag gtgaactgtg aggagagaaa 1200
   cattacagga ttagaaaatt tcactctgaa aattttaaat atgtcacagg accttatgga 1260
   ttttctgaac ccaaacggta gtgactgtac tctagtcctg ttttacaccc cgtggtgccg 1320
   cttttctgcc agtttggccc ctcactttaa ctctctgccc cgggcatttc cagctcttca 1380
   ctttttggca ctggatgcat ctcagcacag cagcctttct accaggtttg gcaccgtagc 1440
   tgttcctaat attttattat ttcaaggagc taaaccaatg gccagattta atcatacaga 1500
   tcgaacactg gaaacactga aaatcttcat ttttaatcag acaggtatag aagccaagaa 1560
   gaatgtggtg gtaactcaag ccgaccaaat aggccctctt cccagcactt tgataaaaag 1620
   tgtggactgg ttgcttgtat tttccttatt ctttttaatt agttttatta tgtatgctac 1680
   cattcgaact gagagtattc ggtggctaat tccaggacaa gagcaggaac atgtggagta 1740
   gtgatggtct gaaagaagtt ggaaagagga acttcaatcc ttcgtttcag aaattagtgc 1800
   tacagtttca tacattttct ccagtgacgt gttgacttga aacttcaggc agattaaaag 1860
   aatcatttgt tgaacaactg aatgtataaa aaaattataa actggtgttt taactagtat 1920
   tgcaataagc aaatgcaaaa atattcaata gatgcactat tcttgttttt actgcatgaa 1980
   cgtaatccag tatttggaaa gtaatccagt ttgaaatgtg aagatgtatt ccggcagaat 2040
   agtgagtaga atgacatgct tactatacag aaggcaaaaa taggactctc aggtaatagt 2100
   ttaaggaaac ccttgattcc ttatatatgt ttaagaaggt tagttttctg tttctttgca 2160
   gtttttcttc tagagtccat agcaggaaag tatgtaacca gaattggtta gtgtgacccc 2220
   ctcaagtagc aagtgatgga aaataagagt caaatacctt gatgtttgtg atctctaact 2280
   caaaaaattt gaagtgtttt aagttgtttc tgggtaaggg agatgttagg agaaaggaaa 2340
   tgctgtaact aaagctcaat tattatcagt tctatgctaa cgtatacatt ttaatcatag 2400
   ttacctaagc agcatgcatt aattgaacct taaaatgttc ccagcaggct gggcgcagtg 2460
   gctcatgcct gtaatcccag cactttggga ggccaaggcg ggaggatcac ttgaggttag 2520
   gacttttgag accagcctgg ccaacttggt gaaaccctct ctctactaaa aatacaaaaa 2580
   attagctggg cgtggtggcg ggcacctgta atcccagcta cttgggaaag ctgaggcatg 2640
   agaatcactt gaacccggga ggcagaggtt gcagtgagct gagatcatgc cactgcactc 2700
   cagcctgtgc aaccagagcg aaactccatc tcaaaaacaa acaaaaagaa gtattgaagt 2760
   gtaaaacact gttcttgccc acaagttgat tctagtctag ttcaagacac atgtttagta 2820
   aagagacaac atgtaattta aacaaacttt tttttttttt ttagacaaag tctcactctg 2880
   tcacctaggc tggagtgcaa tggtgcaatc ttggctcact gcaacctctg cttcccgggt 2940
   tcaagcgatt ctccagcctc agcctcctga gtagctggga ctatgggcct gtgccatcac 3000
   acccagctaa tttttctatt tttagtagag atggggtttt gccatgttgg ccaggctggt 3060
   ctcaaactgc tgacttcagg tgatccaccc gcctcagcct cccaaagtgt tgggattaca 3120
   ggtgtgagcc actgcgcctg gcctaaacaa actttttgaa aagctgtttc taaaagattc 3180
   cttaaattca gatatgacag ctaattacct catcataaat tacttttata ctaattgttt 3240
   ccagggtttt agagtagttg aatgtttatt tcacaaggca ccctaaattc tatagaaata 3300
   aaacctcaga tgagtctcct tcttagagtg ttacaatgaa tgggagttta caacttttat 3360
   gtgtcatgtt tccaacagct gtgtttgggg tggtcactgg caggagggga ccgtatctca 3420
   gaatggcaca ttatttctat tttacacatg agcaaattga ggcatagaga gttagataac 3480
   ttgcccaggt tacacagatt gtaagttgat gaagctggga tttgaatctt cacatgtgtg 3540
   tacttataaa tacaaatgta aggaaaactc tagtgagtcc acctcttata ttgagttatt 3600
   actgtgtgag tgccaagtac tgttttagat gctttacata tactattttg tttaatatcc 3660
   tttttaaaga ataaactagg ttggtgcag 3689
<210> 16
   <211> 1608
   <212> DNA
   <213> Homo sapiens
<400> 16
   agttcgccgc ttgcaccggg accgatgcca tctgagacgc acgcgatgct ggcgacgctg     60
   gcgagggtgg cagctctgcg cagaacctgc ctcttctccg gccggggcgg cgggaggggg    120
   ctgtggactg gccgcccgca gtcagatatg aacaatataa agccattgga aggggtaaaa    180
   attctggatc taacaagagt cctggcggga ccttttgcta ctatgaattt aggagatctt    240
   ggagcagaag ttataaaagt ggagagacca ggagctggtg atgatacacg aacttggggg    300
   ccaccttttg ttgggacaga aagtacatat tatctcagtg ttaaccgaaa taaaaaaagt    360
   attgctgtta atatcaagga tccaaaaggg gtgaaaatca tctattgttc catcacaggg    420
   tatggtcaga caggtccaat ttctcagcga gctggttatg atgctgttgc ctcggctgtt    480
   tctggtctga tgcacatcac agggcctgag aatggagatc cagttcgccc aggagtagct    540
   atgactgatc ttgccactgg cctgtatgca tatggagcta ttatggctgg attgatacaa    600
   aaatacaaaa ctgggaaagg actgttcatt gattgtaacc tgctgtcatc ccaggtggcg    660
   tgtttgtctc acatagctgc aaattatctt attggtcaaa aggaagcaaa acgttggggt    720
   acagctcatg gcagtatcgt tccttaccag gcttttaaaa ccaaggatgg ctatattgta    780
   gttggagcag gaaataacca gcagtttgcc accgtctgca agatcttgga tttgcctgag    840
   ttgattgata attccaagta taaaactaac caccttcggg tacacaatag aaaagagctt    900
   attaaaatat tatctgaacg gtttgaagaa gaactgacca gcaagtggtt atatcttttt    960
   gaaggcagtg gagtcccgta tggcccaatc aacaacatga agaatgtatt tgcagaacct   1020
   cagaacgctg tctctggctt ccaaagcctg ctgcattcct tggcccatgg ccccttcctt   1080
   catcttcaag gatcagcaag ggtattacac aatggcctcg ttatggagat ggagcatcca   1140
   actgtgggga agatttccgt cccaggccca gctgtgagat acagtaagtt caagatgtca   1200
   gaggccaggc cgccccccct gctcgggcag cacacaacgc acatcctgaa ggaggtcctg   1260
   agatacgatg acagggccat cggggagctg ctcagcgctg gagtggtgga ccaacatgaa   1320
   actcactgac aaaggaaaag ggctcttcct cataacctcg atccgaatac actggcaaag   1380
   gcaacacttt gcttggaccc ttctccccag ttctgatacc actaagaaga agatttagag   1440
   taactccaga tttcttacat ggcatctcca gaatggctct ggtattaatg aatctagtgc   1500
   cttttaaatg tatcccacgt tttgttccct accatctttt ttttcagatg atgatttcat   1560
   tatggatttg tgggattttt aaaaataaag atttaatttt tttcctgg                1608
<210> 17
   <211> 727
   <212> DNA
   <213> Homo sapiens
<400> 17
   gaacgagggt cctagctgcc gccacccgaa cagcctgtcc tggtgccccg gctccctgcc      60
   ccgcgcccag tcatgaccct gcgcccctca ctcctcccgc tccatctgct gctgctgctg     120
   ctgctcagtg cggcggtgtg ccgggctgag gctgggctcg aaaccgaaag tcccgtccgg     180
   accctccaag tggagaccct ggtggagccc ccagaaccat gtgccgagcc cgctgctttt     240
   ggagacacgc ttcacataca ctacacggga agcttggtag atggacgtat tattgacacc     300
   tccctgacca gagaccctct ggttatagaa cttggccaaa agcaggtgat tccaggtctg     360
   gagcagagtc ttctcgacat gtgtgtggga gagaagcgaa gggcaatcat tccttctcac     420
   ttggcctatg gaaaacgggg atttccacca tctgtcccag cggatgcagt ggtgcagtat     480
   gacgtggagc tgattgcact aatccgagcc aactactggc taaagctggt gaagggcatt     540
   ttgcctctgg tagggatggc catggtgcca gccctcctgg gcctcattgg gtatcaccta     600
   tacagaaagg ccaatagacc caaagtctcc aaaaagaagc tcaaggaaga gaaacgaaac     660
   aagagcaaaa agaaataata aataataaat tttaaaaaac ttaaaaaaaa aaaaaaaaaa     720
   aaaaaaa                                                               727
<210> 18
   <211> 1792
   <212> DNA
   <213> Homo sapiens
<400> 18
   gtggtgcggg gtggggcctg ggcgagtcac gtggggacgg tgcgcgctca gtgcggctgc      60
   gccggccggt agctgcagct ggagcagtgg cgtttggagg agactcggat ataccttctc     120
   agaagctgca caggaggaaa gcagtgacaa agaaagaagt tgtcattctt tgcacgaaac     180
   tggatggctt ctacagggag ccaggcctct gatatagacg agatttttgg attcttcaac     240
   gatggcgaac ctcccaccaa aaagcccagg aagctgcttc caagcttaaa aactaagaag     300
   cctcgagaac ttgtgctagt gattggaaca ggcattagtg ctgcagttgc gccccaagtt     360
   ccagccctca aatcctggaa ggggttaatt caggccttac tggatgctgc cattgatttt     420
   gatcttttag aagatgagga gagcaaaaag tttcagaaat gtctccatga agacaagaac     480
   ctggtccatg ttgcccatga ccttatccag aaactctctc ctcgtaccag taatgttcga     540
   tccacatttt tcaaggactg tttatatgaa gtatttgatg acttggagtc aaagatggaa     600
   gattctggaa aacagctact tcagtcagtt ctccacctga tggaaaatgg agccctcgta     660
   ttaactacaa attttgataa tctcttggaa ctgtatgcag cagatcaggg gaaacagctt     720
   gaatcccttg accttactga tgagaaaaag gtcctcgagt gggctcagga gaagcgtaag 780
   ctgagcgtgt tgcatattca cggagtctac accaacccta gtggcattgt ccttcatccg 840
   gctggatatc agaacgtgct caggaacact gaagtcatga gagaaattca gaaactctac 900
   gaaaacaagt catttctttt cctgggctgt ggctggactg tggatgacac cactttccag 960
   gcccttttct tggaggctgt caagcataaa tctgacctag aacatttcat gctggttcgg 1020
   agaggagacg tagatgagtt caaaaagctt cgagaaaaca tgctggacaa ggggattaaa 1080
   gtcatctcct atggagatga ctatgccgat cttccagaat atttcaagcg actgacatgt 1140
   gagatctcca caaggggtac atcagcaggg atggtgagag aaggtcagct aaatggctca 1200
   tctgcagcac acagtgaaat aagaggctgt agtacatgag cgagctagag aaatcaccac 1260
   cgtttagacc aagctgtaag gccctactac agacagtgtt taacaagtaa acttacaaga 1320
   acccaacaca attcccagaa agtaacaata gccagaggtt gaagggcggg gtagaagagg 1380
   ggggaatgtt gcagcgtaat ccttcatacc acctggttct tgatattctg ccgcctgttc 1440
   aagttcaaga ataaaagcga cagcaggacc caaatgcagc tcccaaccca ctccccaggc 1500
   tagacatgct tgtgtccaca cagcacacca atgtgatact tccactgacc ggctgcagct 1560
   ctgcatgaag gactcggggt ctggatgcca tggaatcact gtggctcttg ttgcagtttt 1620
   gtactctata cttggttttt caattaagct taatggcttt tttaaaacat gacttgaagc 1680
   tctagttttc tagatctttt acagtgtaca gtattttaca taactaagct gtattaaaag 1740
   cttgttcatt taaaaaaaaa aaaaaaaaaa aaaaaaaaaa aaaaaaaaaa aa 1792
<210> 19
   <211> 2892
   <212> DNA
   <213> Homo sapiens
<400> 19
   gtctgaagcg gcactggaga cccaggaaaa atctcgctga atccgcctgc cccagcagcg 60
   gcctgatctg ggttccgctg attcctttcg taaccgcacc acacccgaga tgaatctacg 120
   ttctgtattt actgtagaac aacaaaggat tttacagcgt tattatgaaa atggaatgac 180
   aaatcaaagt aaaaattgct ttcagctcat attacagtgt gcacaggaga ctaagctgga 240
   cttcagtgta gtcaggacgt gggttggcaa taagagaaga aagatgagta gtaagaactc 300
   tgaatctgga acagcaacaa caggaacctc tttgtcagct ccagacatca cagtcagaaa 360
   tgtggttaat attgctcgac cctcaagcca gcagtcttct tggacatctg ccaataatga 420
   tgtcattgta actggtatat acagtccagc cagttcatca agtaggcaag gaacaaacaa 480
   acatacagac acacaaatta cagaagcaca taaaatccct attcagaaaa cagccactaa 540
   aaatgatact gagtttcagt tacacattcc tgtccaaaga caagtagcac actgtaaaaa 600
   tgcttcccta ctcctaggtg aaaaaacaat tattttgtca agacagacaa gtgtgctaaa 660
   tgctggaaac tcagtattca atcacgcaaa gaaaaactat ggaaactctt cagtacaagc 720
   ttctgaaatg acagtacctc aaaagccttc tgtgtgccac cgaccttgta aaattgaacc 780
   agttgggatt caaaggtcat ataagcctga acacacaggc ccagcattac ataacttatg 840
   tgggcaaaag ccaactatta gagaccctta ctgtagaaca caaaacttgg aaatccgtga 900
   agtgttttca ttggcagtta gcgattaccc ccagagaatt ctgggaggaa atgccccaca 960
   gaagcctagc tcagcagaag gaaattgttt gtccattgca atggagactg gagatgctga 1020
   ggatgaatat gccagagagg aagagctggc atcgatgaga gcacagatac caagctattc 1080
   gagattttat gaaagtggca gttcccttcg agctgagaac caaagtacaa ccttgcccgg 1140
   accaggaaga aatatgccaa attcacaaat ggtgaatatt agagatatgt cagacaatgt 1200
   actgtatcaa aacagaaact accatttgac accacggacc tcattacata cagcatctag 1260
   tacaatgtac agtaatacca atccattacg gagtaatttt tctcctcatt ttgcatcatc 1320
   aaaccaattg agattatcac aaaaccaaaa caattaccag atttcaggaa accttactgt 1380
   gccttggatt acagggtgtt ctagaaaaag agcactacag gaccgcactc agttcagtga 1440
   ccgagactta gccaccctta agaagtattg ggacaatggc atgaccagcc tgggctctgt 1500
   ttgtagagag aaaattgaag ctgtggcaac tgaattaaat gttgactgtg aaatagttcg 1560
   gacttggatt gggaatcgaa gaaggaaata tcgtttaatg gggattgaag ttccacctcc 1620
   aagaggaggc cctgctgatt tctctgagca gcctgagtct ggttctttat ctgcactcac 1680
   accaggagag gaagctgggc ctgaagtagg agaggataat gacagaaatg atgaagtatc 1740
   catctgtttg tctgaaggaa gctctcaaga agagcccaat gaagttgttc cgaatgatgc 1800
   aagggctcat aaggaagagg accaccatgc agtaaccaca gataatgtga aaatagaaat 1860
   tattgatgat gaagaaagtg acatgataag taattctgaa gtagaacaag taaactcttt 1920
   cttggattat aagaatgaag aagtcaaatt cattgaaaat gagctcgaga ttcaaaagca 1980
   aaaatacttt aaacttcaga cttttgttag aagcttgata ttagcaatga aagctgatga 2040
   taaggaacaa cagcaggcac tgctgtcaga tttacctcct gaattagagg aaatggattt 2100
   caatcatgcc tcactggagc ctgatgatac ctcattcagt gtatcttctt tgtcagagaa 2160
   aaatgtctca gaaagtttgt gatttcagtt ggagggaata tatgatacag tcttttggct 2220
   tcgtaacagg tgtgcatttc aagataactg cattctgttg ccctggtatt ctttagttgg 2280
   gaaaacacat tgttgaaacg gacgtattct gtgaagaatg tacaagatat aatggctaca 2340
   gtgcaacaaa aatgtaggtg aaatttaaaa gcattgtttg agagagtatt tttttaactg 2400
   atggaactct ggaaaaaaat tatatttaag tttcagcagt ttaaccctga aattcattat 2460
   gtctaatttc taaccagaga caaaataact aaagacattt cagcattgct tatcaagttg 2520
   ctacagcttg attagtcttg tttttgtagc cattacatct tctttcttct tctctccttt 2580
   tcctatcatc cacttacact ttttctcagg aaagtggact gaacatttaa aacaaaactt 2640
   taaaaaatta tttaactcat tatttaatga gttctctgat ttagttttta acccctatga 2700
   aaatttgact taaactaatg actgaaaatt aaatgattac aggtatgtaa ttgtaaattg 2760
   ctggtgttct tctattatct aacccaaata tttgtgtggg ggtggggaag cacaatggaa 2820
   aggtaattta accaacataa cgtcaaataa attacgaagt gtacagaaac gaaaaaaaaa 2880
   aaaaaaaaaa ca 2892
<210> 20
   <211> 2823
   <212> DNA
   <213> Homo sapiens
<400> 20
   aacgaacaga agggcgagag aattggcagg atccgtctcc tacctcttcc taggcccaca 60
   gccagtgcct ttggagtact gaggcgcgca cagagtcctt agcccggcgc agggcgcgca 120
   gcccaggctg agatccgctg cttctgtgga agtgagcatg gttgggcagc gggtgctgct 180
   tctagtggcc ttccttcttt ctggggtcct gctctcagag gctgccaaaa tcctgacaat 240
   atctacactg ggtggaagcc attacctact gttggaccgg gtgtctcaga ttcttcaaga 300
   gcatggtcat aatgtgacta tgcttcatca gagtggaaag tttttgatcc cagatattaa 360
   agaggaggaa aaatcatacc aagttatcag gtggttttca cctgaagatc atcaaaaaag 420
   aattaagaag cattttgata gctacataga aacagcattg gatggcagaa aagaatctga 480
   agcccttgta aagctaatgg aaatatttgg gactcaatgt agttatttgc taagcagaaa 540
   ggatataatg gattccttaa agaatgagaa ctgtgatctg gtatttgttg aagcatttga 600
   tttctgttct ttcctgattg ctgagaagct tgtgaaacca tttgtggcca ttcttcccac 660
   cacattcggc tctttggatt ttgggctacc aagccccttg tcttatgttc cagtattccc 720
   ttccttgctg actgatcaca tggacttctg gggccgagtg aagaattttc tgatgttctt 780
   tagtttctcc aggagccaat gggacatgca gtctacattt gacaacacca tcaaggagca 840
   tttcccagaa ggctctaggc cagttttgtc tcatcttcta ctgaaagcag agttgtggtt 900
   tgttaactct gattttgcct ttgattttgc ccggcccctg cttcccaaca ctgtttatat 960
   tggaggcttg atggaaaaac ctattaaacc agtaccacaa gacttggaca acttcattgc 1020
   caactttggg gatgcagggt ttgtccttgt ggcctttggc tccatgttga acacccatca 1080
   gtcccaggaa gtcctcaaga agatgcacaa tgcctttgcc cacctccctc aaggagtgat 1140
   atggacatgt cagagttctc attggcccag agatgttcat ttggccacaa atgtgaaaat 1200
   tgtggactgg cttcctcaga gtgacctcct ggctcacccc agcatccgtc tttttgtcac 1260
   tcatggtggg cagaacagcg taatggagac catccgtcat ggtgtgccca tggtgggatt     1320
   accagtcaat ggagaccagc atggaaacat ggtccgagta gtagccaaaa attatggtgt     1380
   ctctatccgg ttgaatcagg tcacagccga cacactgaca cttacaatga aacaagtcat     1440
   agaagacaag aggtacaagt cggcagtggt ggcagccagt gtcatcctgc actctcagcc     1500
   cctgagcccc gcacagcggc tggtgggctg gatcgaccac atcctccaga ctgggggagc     1560
   gacgcacctc aagccctatg tcttccagca gccttggcat gagcagtacc tcattgatgt     1620
   ctttgtgttt ctgctggggc tcactctggg cactatgtgg ctttgtggga agctgctggg     1680
   tgtggtggcc aggtggctgc gtggggccag gaaggtgaag aagacatgag gctaggtgta     1740
   gccttgggtg aggggagggc atccctggtc ctttgaaggt tctccccacc ccagcacacg     1800
   ccacccctct gttctctctt cagctccacc cgccactgat cctgcaactt gcttctttct     1860
   attctctgcc tctgtttaga aatcttcaca caccactgag gcttcttgac ttgccccttg     1920
   tgacttgaaa ccccagctca gatacaaatt ttcacctgcc agccctgcct cctcctttct     1980
   cccttttcct agacacagga ctctgacaac ttcatcctcc ttgtttagat gacttcccag     2040
   tttccagtcc ccatttctcc ttctatcact tttcataaaa aaactcagga aatatttgac     2100
   atatcttcca tttcaaattc ttccatttta tgcagatatc ttgcccttcc tataagctct     2160
   cctcaaagct caggaaacct ggtctgctct cctgcattta gggaaggaga acccctgcca     2220
   agacctttgc tcactgcctg agaccccttc cttagagagc acctcctttg ctggtcagac     2280
   atggagcctg cagttggtca cagatgatac tgctttattt cagtttttac agttgccttc     2340
   ttaagattcc cgtcttataa atggagtaca gggaacctca agtagtgaag tggaaatcca     2400
   tgtgtaaggc tttgtggctt caggtaccag tggctaaggt agttttaaag actttgttga     2460
   ttttagaaaa agtccatctt ccatccccta catggcagtt aatacccttc tatatggtaa     2520
   aaccttagag attaccttaa tctgctagga acagaagcaa gaaaaaccat ggcgtaaaca     2580
   cccccagagt ttttgttcat ttgtttcatc tttcttgata aagcccgaag gtagcccatt     2640
   cagggctgtt gtggttggtt gctccatcat gtcatcaata gcccatatct tttctttttt     2700
   atcttcctta gtataacacc aaactacctc tctgatagct ggtgttcatg aaatatttta     2760
   ccttcaaatg attgtacctt tttatttgct ttagagttct gaaataaaat gaaattccac     2820
   tgt                                                                   2823
<210> 21
   <211> 2785
   <212> DNA
   <213> Homo sapiens
<400> 21
   ttactttctt actcttgggt cagttcctgc tacagctatc ccaccatgga cttcttgatg     60
   agtggcctgg cagcctgcgg ggcctgtgta ttcaccaatc ccctggaggt ggtgaagacc 120
   aggatgcagt tgcaaggaga actgcaggcc cctggcacat accagcggca ctaccgaaat 180
   gtcttccatg ccttcatcac catcggcaag gtggatggcc ttgctgccct gcagaaaggc 240
   ctggcccccg ccctcttgta ccagttcctg atgaatggca tccgactggg cacctatggg 300
   ctggctgagg ctgggggcta cctgcacaca gccgaaggca cccacagtcc tgcccgcagc 360
   gcagcagctg gggccatggc tggggtcatg ggagcctact tggggagccc catctacatg 420
   gtgaagacac acctgcaggc acaggcagcc tcagaaattg ctgtagggca ccagtataag 480
   catcagggca tgtttcaggc gctaaccgag attggccaga aacatggtct ggtggggtta 540
   tggcgtgggg ctctgggcgg cctgccccga gttatcgtcg gttcctccac ccagctgtgc 600
   accttctcat ccaccaagga cctcctgagc cagtgggaga tctttcctcc ccagagctgg 660
   aagttggcgc tggtggctgc catgatgagt ggcattgcag ttgtcttggc catggcaccc 720
   tttgatgtgg cctgcacaag gctctacaac cagcccacag atgcacaggg caagaaccga 780
   gtccccaagt tctcagcaac atcatgcagt gcttcagcgc ctttactggg gaccaaagat 840
   gttaggatcg tgaagggtca gactggtcac cagctgttca aagtcaccca ggctccaggg 900
   tgcagcccga ttctcctggg aacccagtca tgagataccc gcccgttcct tcctccttac 960
   gggggctgat tgaaggtaag caagagatca gtctggtact ggggcagcct cagcaaggcc 1020
   tgatgaagtg tcacgtcctt tgctacctaa ggggatcaga gtcaggatgc ttacctggat 1080
   ctctgcgtcc ccgctaccgc ccttccagga cattacacac tctcgggccc tcacctgctg 1140
   gttttcctta gctatctgct gcttgccaga gaggacccag gcttcttgac agcggcccct 1200
   cagggccagg ttctccaaac tgagaggctg aacagactcc acatggacag ccctagcccc 1260
   ctgcacgcca ccaacatcct caaagtggta cctggagtag ggaagcgggg tcaggaaggg 1320
   acatctctgg tcgcctcccc gggagcatct ttaagttgca ggcctggctc ccggcccgtg 1380
   cgtggtctca ccgcccggat taaccgtcgg ccgcgggccc cgcccactgg tccgaccccg 1440
   cccctgccat tggccagccg ggggccattc cctcaccgcg cagccgcttc gccccgtacg 1500
   tgggcctgca gctcgagaag ttccactatc aggctctggt ccgtcacggg atggcagaaa 1560
   acttcttgat tgtccgggac cggtcggagg tcgctggagt gaggcgaggc agaccctggt 1620
   tgaccccggt ctcttcccac aacccaccca gtcagccgcc cctgggcgcc ccggccttct 1680
   caccttacgt caatggcccc catggggagg atggcggaaa aggcgccccc gaacagcggg 1740
   cagtctctcg tgggctccat gggtctgagg ctgggttatg ggcattagaa aggtttaaaa 1800
   gaagtccaca ttgggtcacg tggcatcggc tccctgggtt tcgcagctgc caccgccacc 1860
   cgggtctggc ttagctcccg gatcctttaa gatttggctc cgccctgcgc tctcgttggc 1920
   caatcggcgc caagaccctg ggggcggggg tcagcggctt tgctccggaa gccgcgctct 1980
   taggagtgcg cctggcggga cggtggggtc tgccttgggg ttggacttag ggttagggtc 2040
   tatgtcgccc ccggtggtca cgagcatgct cccgccagcc tcctcttgat tgattgattg 2100
   attgattgat tgacaggtct cgctgtgtcg cccaggctgt aatgcagtgg cgcgatcacc 2160
   gctcactgca gtctccacct ccaggactca agtgatactc ctgcctcagc ctcttgagtg 2220
   gctcctgctc ccgccaccac gcccggctaa atttttaatt acttgtagaa atggggcggg 2280
   gtggggagtg tctcccaatc ttgcccaggc tggcctcaaa ctcctggact caagcgatcc 2340
   tcccgcctcg gctccctaag tgctggaatt acaggcgaga gccacagcgc ctggcctcct 2400
   cgtccttttc aagtggtcac accttccaag aaggtcgcct gagaccctcc tgaggggcag 2460
   gtgctcgtca aggggcccca cgtctaggta gggtctcttg agtcaggaaa gcatagtttc 2520
   acggtgttct gggcctctga aagaagaatg ggctgccacc cagaccgcta attcatagat 2580
   ctgctgctgg gaattagggg aacaatgcgt ccaggttagg attcaggtct cacatcttcc 2640
   tgaaagtgag agccaacctg ggtattagaa atctaattcc tctcaaatat ttgccttggg 2700
   aatgctcccg ggcaggtgca accccacccc cagctccaca cacctggaca gaagcacaga 2760
   ttaaaccctc ttttgtttgt ctttt 2785
<210> 22
   <211> 2414
   <212> DNA
   <213> Homo sapiens
<400> 22
   gaagatgcag tttctccgta tccgcaggct tctttcgctg gcgccattac ctgagttctc 60
   ctccagcgtt tccgcaccct ctccgattag cggtcccagg agtttccagg gtaaccgcgc 120
   agtagggcgg atctcattag gcggaaagcg aaacccggaa gtgacgctct taccgggtgt 180
   cagcagcgag agggttcgaa gatggcggcg cgcaagggtc ggcgtcgcac gtgtgaaacc 240
   ggggaaccca tggaagccga gtccggcgac acaagttccg agggcccggc ccaggtctac 300
   ctgcccggcc gggggccgcc gctacgcgaa ggggaggagc tggtcatgga cgaggaggcc 360
   tatgtgctct accaccgagc gcagactggc gccccctgtc tcagctttga catagtccgg 420
   gatcacctgg gagacaaccg gacagagctt cctcttacac tttacttgtg tgctgggacc 480
   caggctgaga gcgcccagag caacagactg atgatgcttc tgatgcacaa tctgcatggg 540
   acaaagcccc caccctcaga gggcagtgat gaagaagaag aggaggaaga tgaagaggat 600
   gaagaagagc ggaaacctca gctggagctg gccatggtgc cccactatgg tggcatcaac 660
   cgagttcggg tgtcatggct gggtgaagag cctgtggctg ggtgtggtgc agagaagggc 720
   caggtggagg tgtttgcgct gcggcggctt ctgcaggtgg tggaggagcc ccaggccctg 780
   gcagccttcc tccgggatga gcaggcccaa atgaagccca tcttctcctt cgctggacac 840
   atgggcgagg gctttgccct tgactggtcc ccccgggtga ccggtcgcct gctgaccggt 900
   gactgtcaaa agaacatcca cctctggaca cctgcggacg gcggctcctg gcacgtggac 960
   cagcggccat tcgtgggcca cacacgctct gtggaggacc tgcagtggtc accgactgag 1020
   aacacggtgt ttgcctcctg ctcagctgac gcctccatcc gcatctggga catccgggca 1080
   gcccccagca aggcctgcat gctcaccaca gccaccgccc atgatgggga cgtcaatgtc 1140
   atcagctgga gccgccggga gcccttcctg ctcagtggcg gggatgatgg ggccctcaag 1200
   atctgggacc ttcggcagtt caagtctggt tccccagtgg ccaccttcaa gcagcacgtg 1260
   gcccccgtga cctccgtcga gtggcacccc caggacagcg gggtctttgc agcctcgggt 1320
   gcagaccacc agatcacaca gtgggacctg gcagtggagc gggaccctga ggcgggcgac 1380
   gtggaggccg accccggact ggccgacctc ccgcagcagc tgctgttcgt gcaccagggc 1440
   gagaccgagc tgaaggagct gcactggcac ccgcagtgcc cagggctcct ggtcagcacg 1500
   gcgctgtcag gcttcaccat cttccgcacc atcagcgtct gaggcgtccc actggctctg 1560
   atcttgcttc ctgcttggaa actgaagtcg aattgggctc ccctggaagg ggttcattca 1620
   ggtctgttga ctgagactgg ccggcctgtg ggctgccgtg atggattctg tttgacgtat 1680
   tgttctctag aaggcctggc tctgatccag tgacccctct caccaaagaa ctcggtttaa 1740
   ccagggctct gtaagaccac tcccacccag agacttgtgt ggcctggtgt ggcctgtgtg 1800
   tcggattcct tcctgtcagc tgtgacccat ttgacctgtg tccccagaac ccagtttttt 1860
   gtttgtttgt ttgagacgga gtcttggtct gtcgcccagg ctggagtgca gtagcacgat 1920
   cttggctcac tgcaacctcc gcctcctggg ttaaagtgat tctctcagct cagtctccca 1980
   ggtagctggg attacaggca tgtgccacca caccccgtta atttttgtat ttttagtaga 2040
   gacggggttt caccatgttg gccaggctgg tctcaaattc ttgatctcaa gtgatctgtc 2100
   cgccccggcc tcccagagtg ctgggttggg attacaggcg tgagccaccg cgtccggctc 2160
   aggacccagt tttggctgct ggttcccagc aggggactcg ggggatatac agtggctgca 2220
   ccaaattgga ggtgtgggtt cctccaacac aatttgcttc tgcccgttgt cttcctgcca 2280
   gctgggtttg gccaggattt ctccgtgtgg gggctacatg cgaccctctc ccctcctccc 2340
   tgactttaga ggctggtgct gtgtcgggag gaaggtcagg gctcctgagc agcaataaag 2400
   gaccaagaag aggc 2414
<210> 23
   <211> 4700
   <212> DNA
   <213> Homo sapiens
<400> 23
   atggccccct cggcctgggc catttgctgg ctgctagggg gcctcctgct ccatgggggt 60
   agctctggcc ccagccccgg ccccagtgtg ccccgcctgc ggctctccta ccgagacctc 120
   ctgtctgcca accgctctgc catctttctg ggcccccagg gctccctgaa cctccaggcc 180
   atgtacctag atgagtaccg agaccgcctc tttctgggtg gcctggacgc cctctactct 240
   ctgcggctgg accaggcatg gccagatccc cgggaggtcc tgtggccacc gcagccagga 300
   cagagggagg agtgtgttcg aaagggaaga gatcctttga cagagtgcgc caacttcgtg 360
   cgggtgctac agcctcacaa ccggacccac ctgctagcct gtggcactgg ggccttccag 420
   cccacctgtg ccctcatcac agttggccac cgtggggagc atgtgctcca cctggagcct 480
   ggcagtgtgg aaagtggccg ggggcggtgc cctcacgagc ccagccgtcc ctttgccagc 540
   accttcatag acggggagct gtacacgggt ctcactgctg acttcctggg gcgagaggcc 600
   atgatcttcc gaagtggagg tcctcggcca gctctgcgtt ccgactctga ccagagtctc 660
   ttgcacgacc cccggtttgt gatggccgcc cggatccctg agaactctga ccaggacaat 720
   gacaaggtgt acttcttctt ctcggagacg gtcccctcgc ccgatggtgg ctcgaaccat 780
   gtcactgtca gccgcgtggg ccgcgtctgc gtgaatgatg ctgggggcca gcgggtgctg 840
   gtgaacaaat ggagcacttt cctcaaggcc aggctggtct gctcggtgcc cggccctggt 900
   ggtgccgaga cccactttga ccagctagag gatgtgttcc tgctgtggcc caaggccggg 960
   aagagcctcg aggtgtacgc gctgttcagc accgtcagtg ccgtgttcca gggcttcgcc 1020
   gtctgtgtgt accacatggc agacatctgg gaggttttca acgggccctt tgcccaccga 1080
   gatgggcctc agcaccagtg gggggcctat gggggcaagg tgcccttccc tcgccctggc 1140
   gtgtgcccca gcaagatgac cgcacagcca ggacggcctt ttggcagcac caaggactac 1200
   ccagatgagg tgctgcagtt tgcccgagcc caccccctca tgttctggcc tgtgcggcct 1260
   cgacatggcc gccctgtcct tgtcaagacc cacctggccc agcagctaca ccagatcgtg 1320
   gtggaccgcg tggaggcaga ggatgggacc tacgatgtca ttttcctggg gactgactca 1380
   gggtctgtgc tcaaagtcat cgctctccag gcagggggct cagctgaacc tgaggaagtg 1440
   gttctggagg agctccaggt gtttaaggtg ccaacaccta tcaccgaaat ggagatctct 1500
   gtcaaaaggc aaatgctata cgtgggctct cggctgggtg tggcccagct gcggctgcac 1560
   caatgtgaga cttacggcac tgcctgtgca gagtgctgcc tggcccggga cccatactgt 1620
   gcctgggatg gtgcctcctg tacccactac cgccccagcc ttggcaagcg ccggttccgc 1680
   cggcaggaca tccggcacgg caaccctgcc ctgcagtgcc tgggccagag ccaggaagaa 1740
   gaggcagtgg gacttgtggc agccaccatg gtctacggca cggagcacaa tagcaccttc 1800
   ctggagtgcc tgcccaagtc tccccaggct gctgtgcgct ggctcttgca gaggccaggg 1860
   gatgaggggc ctgaccaggt gaagacggac gagcgagtct tgcacacgga gcgggggctg 1920
   ctgttccgca ggcttagccg tttcgatgcg ggcacctaca cctgcaccac tctggagcat      1980
   ggcttctccc agactgtggt ccgcctggct ctggtggtga ttgtggcctc acagctggac      2040
   aacctgttcc ctccggagcc aaagccagag gagcccccag cccggggagg cctggcttcc      2100
   accccaccca aggcctggta caaggacatc ctgcagctca ttggcttcgc caacctgccc      2160
   cgggtggatg agtactgtga gcgcgtgtgg tgcaggggca ccacggaatg ctcaggctgc      2220
   ttccggagcc ggagccgggg caagcaggcc aggggcaaga gctgggcagg gctggagcta      2280
   ggcaagaaga tgaagagccg ggtgcatgcc gagcacaatc ggacgccccg ggaggtggag      2340
   gccacgtaga agggggcaga ggaggggtgg tcaggatggg ctggggggcc cactagcagc      2400
   ccccagcatc tcccacccac ccagctaggg cagaggggtc aggatgtctg tttgcctctt      2460
   agagacaggt gtctctgccc ccacaccgct actggggtct aatggagggg ctgggttctt      2520
   gaagcctgtt ccctgccctt ctctgtgctc ttagacccag ctggagccag caccctctgg      2580
   ctgctggcag ccccaaggga tctgccattt gttctcagag atggcctggc ttccgcaaca      2640
   catttccggg tgtgcccaga ggcaagaggg ttgggtggtt ctttcccagc ctacagaaca      2700
   atggccattc tgagtgaccc tcagagtggg tgtgtgggtg cgtctagggg gtatcccggt      2760
   agggggcctg cagggagcca gagggtggaa atggcctcta agctagcacc ccgtaagaag      2820
   agcctacctg accgacttgg ggagggaaca cagaggtgtt gggaaggtgg agcaacaatg      2880
   cacctcccct cctgtcgcgc cgtgatatct tggtggctcc ctgccactgc ccaccgcctc      2940
   ttctccatct gagaatcacg gagaggtgta gataatctag aggcatagac tgctagagcc      3000
   cccagggatc tggggtggtc agggctcagg cttcactttg taaaccaggt gggggcatct      3060
   cacagcctga cttcccttcc ccaggccagg gttgctggga tgcctgcccc tcctgagagg      3120
   accccctccc cattgtcagg ctctccatgt ccacgagcgg ggaggggtgg gttctggggc      3180
   attgttgtcc cttgtgtctg tggactagag atagggtggg ggagctgggg aagggtgcag      3240
   gcgggaagag tgggctgtct ttcccagggt gatgcaagca tgccgcagcc ctggaggctg      3300
   ggaatgtgga ggctctgtga gccctgcagc cctcagaatc agggccaggg atgcagaaga      3360
   ttgagaggat atggagatgg atagagggca ggagaccctt aggatagatt gtgggaccca      3420
   ggcaggaaca ggtgtccaca agaactcagg atggcatcag ttagctcaga agccacctgg      3480
   aagacccagt gtttccatct ctggaatctc tgttttatgc taaatggatt taggaagact      3540
   gtttttcttt taagggggaa acaaggtaga gaaaaggacg aagaagtgta agtcccgctg      3600
   attctcgggg gtaaggctcg gatggcaagg acgcgttctg cctgggcatg taggggaggt      3660
   gtttttgcca tcaccagttt ctcaggctgg ggagcacaga ggggaggagg aggactaaat      3720
   gaaaagttgt tcccagcctg cacatgaaca cattcatgac acacaaaact ggctggaagg      3780
   agataagagc actgggtttg agattccctc cattaaaaca accaagacaa agaaaggagg 3840
   ggaaaaaaag ataaaaagca agccagggtt ccctgcccta ttgaaactca aacccagact 3900
   gccttgggtt ttatctttcc cttacccctg gcacctccag agaactggga cctgaaatag 3960
   tccctccgtt ctcccctttg accatgtaat aaatgaacca gaagcactga gattaaccta 4020
   tcaacgccct gagaagcctt ccagcctgcg gtgctgtctg ctgggaggtc agctggtcaa 4080
   ggcagaggag gagaggagga aaggatgggg gctgaagagc agaagggagg ggagacagag 4140
   gggattaaag aggggaggag agagtgcaga gctccaggaa agggtatcag agctgcagcc 4200
   agctctgccc tctaccctag ggaggccaga aagacacaaa cagccctccg ggcctttacg 4260
   ctggactctg gcttggcagg ctccaggcag ggtcctctgg gaagttactc tagaaaacga 4320
   agggaggagg agcacaagat cctcagcaac gaacacctgc acttagaaaa agtggacagc 4380
   ttctgccaac cacaccctac ccatggtact gtatgctatt aactcctgga aacgccccgt 4440
   aaatgcgagt tgtttttgta tttgtgtgtt gagatgggcc ttgtggtttc tctgtactca 4500
   gagcacattt cttgtaatta ctattgttat ttttattgtc atgactgccc ctgagctctg 4560
   gtgagaaaag ctgaatttac aaggaaaggg atgaagttaa tatttgcatc acataattat 4620
   atcattactg tgtatctgtg tattgtacta aatggactga tgctgcgcac atgagctgaa 4680
   aatgaagagc cctcccatcc 4700
<210> 24
   <211> 3081
   <212> DNA
   <213> Homo sapiens
<400> 24
   gctgcgggct ccgcgcgcgc gggccatgtc cgctttctgc ctgggcttgg tcggccgcgc 60
   ttcagcaccc gccgagccgg acagcgcctg ctgcatggag ctgcccgccg cggccgggga 120
   cgcagtccgg agtcccgccg ccgccgccgc cctcatcttc cccgggggct ccggggagct 180
   agaactggcg ttagaggagg agctggcgct gctggcggcc ggggagcggc cgtccgaccc 240
   cggggaacac cctcaggccg agcctgggtc tctggccgag gggcgcggac cgcagccgcc 300
   gccctcccag gaccccgagc tgctgtcggt gatccgacag aaggagaagg atctggtgtt 360
   ggcggcccgg ctgggtaagg cgctgctcga gaggaaccag gacatgagcc ggcagtacga 420
   gcagatgcat aaggagctga cagacaagct cgagcactta gagcaagaga aacatgaatt 480
   gagaagacga tttgagaacc gagaagggga gtgggaaggc cgagtgtcag agctggagag 540
   tgatgtgaag cagctacagg atgagttgga gaggcagcag attcatctgc gggaagcaga 600
   tcgagaaaaa tcacgggctg tccaggaact gtcggaacag aaccaaaggc tattggatca 660
   gctcagcagg gcatcagaag ttgagagaca actctccatg caggtccacg ccctcagaga 720
   agactttcgg gagaaaaact catcaaccaa ccagcacatt atccggctgg agagccttca 780
   ggccgagatc aagatgctgt cagatcggaa acgggagctg gagcatcgtc tcagcgctac 840
   tttagaggaa aatgacctgc tccaagggac cgtggaggag ctacaggacc gggtgctaat 900
   cctggagagg cagggccatg acaaggacct acagctgcac caaagccagc tggagcttca 960
   ggaggtgcgt ctctcctgcc gacagctgca ggtgaaggtg gaagaactca ctgaggagag 1020
   gagtctgcag agctctgccg ccaccagcac atccctcctg tcagagatcg agcagagcat 1080
   ggaggctgag gagctggagc aggagcgaga gcagctgaga ctgcagctct gggaagccta 1140
   ctgccaggtt cgctatctgt gctcacacct tcgaggcaat gacagtgctg actcagccgt 1200
   ctccacggac tcctccatgg acgagtcttc agaaacctcg tccgccaagg atgtgccagc 1260
   cggcagcttg cgcactgccc tcaatgagct caagagactg atacagagca ttgtggatgg 1320
   catggagccc acggtgacac tgctgagtgt ggagatgact gccctaaaag aggagagaga 1380
   ccgactcaga gtcacttctg aggacaagga gccaaaggag cagcttcaga aggccatcag 1440
   ggaccgcgac gaggccattg caaagaagaa tgctgtggag ctggaacttg ccaagtgcag 1500
   gatggatatg atgtctctga acagccagtt gctggatgcc attcagcaga aactgaacct 1560
   ctcgcagcag ctggaagctt ggcaggatga catgcacagg gtcattgacc ggcagctgat 1620
   ggacacgcac ctgaaagaac ggagccagcc ggctgctgcc ctctgcaggg gccacagcgc 1680
   tgggcggggg gatgagccca gcatcgctga aggcaaacga ctcttctcat tcttcaggaa 1740
   aatttaagtt gggaggagtc aggccaccaa agatgggtgg actggaggca gctggaaagg 1800
   cggtgcaggc aaggcctccc ctgcagcttg cacctcagca gctgccctgc ccctcatgct 1860
   agggccccat gggtccggga gggcctgctc cctttcgtcg gtggggatgg agacctagag 1920
   gtgggggcct gccttggcca ctgaaggctt cccttggccc accgcctggc caagcccacg 1980
   cctgggcttc tccaggacca cgtgcttgag cagggttagg ccacctccca gaggggcccc 2040
   ttggtgttgg gctttgcagc tcacacccaa cagatcgcag cccaccccca ggcactgctg 2100
   cctccttgat tttagcaaat ggggaacaga aggaatggag gcccttctct gcatgcctca 2160
   ggaggcctga gccccagggg cctagacctg tgggggcagc gggccaggcc tgagcctcca 2220
   ttccttcccc agcccctggc ccagggtcaa aggagagatg gcagcccctc ccccgcatgc 2280
   atgcacctca gctggcagga ggccaagcct ctggccgcag ggtctaagag ccggggctta 2340
   cccaagctca gctgaggcca cccgagcccc agggaggaag aaggccctgt ccccctgtcg 2400
   ccactgctct ccctcccagc cttcagtctc tgccccttag cagggcctgg ccaggcagag 2460
   tgttatcacc agtcatctgc aggctttagc catccagccc tttcccctgc tcagggctgg 2520
   ggttggacgg ggtctcctcc tcccacagct ccctcctcca cccctcacat acatacataa 2580
   tttcttggcc tagccaaaca agtccaggcc actgaatggc accagagggg tctgtggtca 2640
   gccaccccac cttgagggca gcacaggcac cacggggtgg aggggagggg gaggctgccg 2700
   gaagcctcca gatgctgcct gcctgcctgc agaagcctgc agtggctgct gctcctgcct 2760
   ctgcagccgc ccctcctcct ccacccaggc cccaactcag aggctccgcg gcccggccag 2820
   ccctcagctg ctcacaaccg attcagtctc cctccctccc tcacgtgggg aaagcacagc 2880
   agggatgcgc ggcaagaatg tacctgtaga tgtgtacata ccacagtgct gtaattttgt 2940
   atgtagcaat catgtaaata catgtatgga ttttataata tacatatata aaaatctata 3000
   aaggcatatt tttagaaaaa cagcacacca ctgcttcttt tgaaaatagt ctgaataaga 3060
   ataaaatgaa tttctacaga g 3081
<210> 25
   <211> 2385
   <212> DNA
   <213> Homo sapiens
<400> 25
   gttagtcgtc gccgtctgag gtgttccctg gctttgtctc gccgtcgttg ccgccccgtc 60
   cctgccttcg tgccccccgc cttggcccct gcccagccgc tctccctgtc ctcctcccct 120
   taaccacccc cacggtttct gccgtcgggg cccgggggcc gggcggatga tgccgtcgga 180
   gagcggagct gagcgcaggg accgggcggc tgctcaggtg gggacggctg cggccacggc 240
   ggtggccacg gcagccccgg caggcggcgg ccccgacccg gaggccttat cggccttccc 300
   cggacggcac ctgagtgggc tgagctggcc acaggtgaag cgactggacg ctcttctgag 360
   cgagccgatt cccattcacg ggcgcggcaa cttccccacg ctgagcgtgc agccccggca 420
   gatcgtgcag gtggtccgca gcaccctgga ggagcaggga ctacatgtgc acagtgtgcg 480
   gctgcatggt tcagctgcca gccacgtgct gcaccctgag agtggcctgg gctacaagga 540
   tctggacctg gtgttccggg tggacctgcg cagtgaggca tccttccagc tgaccaaggc 600
   agtggtgctg gcctgcctac tagacttcct gccggccggt gtgagccggg ccaagatcac 660
   gccactgaca ctcaaggagg catacgtgca gaagctggtg aaagtgtgca cagactcgga 720
   ccgctggagc ctcatctcac tgtccaacaa gagcggcaag aacgtggagc tcaagtttgt 780
   ggactcggtg agacgccagt ttgaattcag catagactcc ttccagatca tcctggactc 840
   cctgttgctc tttggccagt gctcgtccac tcccatgtct gaggccttcc acccaacggt 900
   cacaggcgaa agcctgtacg gggacttcac cgaggccctg gagcacctgc ggcaccgtgt 960
   catcgccacg cgcagtcccg aggagatccg aggtggtggc ctcctcaagt actgccacct 1020
   cctggtgcgg ggcttccggc cccggcccag caccgatgtg cgcgccctgc agcgctacat 1080
   gtgctcccgc ttcttcatcg actttccaga cctggtggag cagcggcgca ccctagagcg 1140
   ctacctggag gcccacttcg gtggggcaga tgcagcccgc cgttacgcct gcctggtgac 1200
   actgcaccgg gtggtcaacg agagcaccgt gtgcctcatg aaccacgagc gccgccagac 1260
   gctggacctc attgccgcac tggcgctgca ggcactggct gagcagggcc cagctgccac 1320
   tgccgccctg gcctggcgcc ctccaggcac tgacggggtt gtgccagcca ctgtcaatta 1380
   ctacgtgacc cccgtgcaac ctctcctggc tcacgcctat cccacctggc tgccttgtaa 1440
   ctgactcaga ccctggccag aagggaaggg actgggcctc acggggtggg gtggggcctc 1500
   caagagtgtg tggaggacat gaccagaggc gcagaatgtg ccaggaggcc cagcactgca 1560
   gggttgggcc tttgattgaa cagaccagac tttcccgagc acgaggcccc tgtgggctta 1620
   atgccagcct ggggctctca gcaggaggac cctttggtta ttgcatcaca cttttggacc 1680
   agcatgccgg tggggtgcag gcgccatttg tcttggaagt caatttcctt caggagacaa 1740
   agcaagtttg gggtggtagc cttaatgccc agcaccttgg attgattccc atggtctaag 1800
   agggtctttt tgactaaggc cagcctgtgg tgatcccagc agctttggag ggcctgcagc 1860
   ccacacactg actgatacat ggtatgttac agggacttgt cactttggat caggctgggc 1920
   cactacaggg cctgatgggc caacttgggt aatcactggg aggagggaaa ctgaggcccg 1980
   aggcagatac ctgggtttgg ggagggctgg gtttagcccg gttctctgaa ctacataccc 2040
   ccagctgtga agccttcctc ttgaggggtt gaggtgggca gtatacagga gccaagcagc 2100
   ccagggtggg tcgcagccca ggccccttcc agggtgccac tcctttcccc cttgtgttgc 2160
   aaggagccct cctgcctggg agcttgttct ttggaaggtg ctgagcctga atttgccaca 2220
   gtcaagtcaa cgttcccacc ccactggctt ggttacaggg ctctcacatg gggtagggga 2280
   gccatactcc cacccccttg cgtattcccc tgggtttgtt gatattttgc actcttaaca 2340
   cctgccaata aagacggtct acactgaaaa aaaaaaaaaa aaaaa 2385
<210> 26
   <211> 2964
   <212> DNA
   <213> Homo sapiens
<400> 26
<210> 27
   <211> 2386
   <212> DNA
   <213> Homo sapiens
<400> 27
   accaagtgag gaaactgggg gacgctgtgg ggaggggcgt ggggctggat cgcgcagcgg        60
   ctgcttcctt taccttcctc ccatggtctc cttccggttc tcgatgcttc tctgagccta       120
   agggtttccg ccactcgttc accctccccc cagctcatga tcttcctccc tcccccgccc       180
   tcctggtcca atctccgatc tgtttagtaa gaaggtgctg ttccgagaag gagaaggaaa       240
   agggcttgac acgtattcac tcggccccgg acgtgggaag caagccgtct ggcttcggcc       300
   tcacatcggt cctgtgctcg ggacggcggc gttggcggac tgatccgcgg cggtgaagag       360
   gcgcctgtgt ctggcagagc tggtgtgaga cgagacaatc ctgccccgcc gccgggataa       420
   tcaagagttt tggccggacc tttgagcata caccgagaga gtgaggagcc agacgacaag       480
   cacacactat ggcgctgaaa cggattaata aggaacttag tgatttggcc cgtgaccctc       540
   cagcacaatg ttctgcaggt ccagttgggg atgatatgtt tcattggcaa gccacaatta       600
   tgggacctaa tgacagccca tatcaaggcg gtgtattctt tttgacaatt cattttccta       660
   cagactaccc cttcaaacca cctaaggttg catttacaac aagaatttat catccaaata       720
   ttaacagtaa tggcagcatt tgtctcgata ttctaagatc acagtggtcg cctgctttaa       780
   caatttctaa agttctttta tccatttgtt cactgctatg tgatccaaac ccagatgacc       840
   ccctagtgcc agagattgca cggatctata aaacagacag agataagtac aacagaatat       900
   ctcgggaatg gactcagaag tatgccatgt gatgctacct taaagtcaga ataacctgca       960
   ttatagctgg aataaacttt aaattactgt tccttttttg attttcttat ccggctgctc       1020
   ccctatcaga cctcatcttt tttaatttta ttttttgttt acctccctcc attcattcac    1080
   atgctcatct gagaagactt aagttcttcc agctttggac aataactgct tttagaaact    1140
   gtaaagtagt tacaagagaa cagttgccca agactcagaa tttttaaaaa aaaaaatgga    1200
   gcatgtgtat tatgtggcca atgtcttcac tctaacttgg ttatgagact aaaaccattc    1260
   ctcactgctc taacatgctg aagaaatcat ctgaggggga gggagatgga tgctcagttg   1320
   tcacatcaaa ggatacagca ttattctagc agcatccatt cttgtttaag ccttccactg    1380
   ttagagattt gaggttacat gatatgcttt atgctcataa ctgatgtggc tggagaattg    1440
   gtattgaatt tatagcatca gcagaacaga aaatgtgatg tattttatgc atgtcaataa    1500
   aggaatgacc tgttcttgtt ctacagagaa tggaaattgg aagtcaaaca ccctttgtat    1560
   tccaaaatag ggtctcaaac attttgtaat tttcatttaa attgttagga ggcttggagc    1620
   tattagttaa tctatcttcc aatacactgt ttaatatagc actgaataaa tgatgcaagt    1680
   tgtcaatgga tgagtgatca actaatagct ctgctagtaa ttgatttatt tttcttcaat    1740
   aaagttgcat aaaccaatga gttagctgcc tggattaatc agtatgggaa acaatctttt    1800
   gtaaatgcaa agctgttttt tgtatatact gttgggattt gcttcattgt ttgacatcaa    1860
   atgatgatgt aaagttcgaa agagtgaata ttttgccatg ttcagttaaa gtgcacagtc    1920
   tgttacaggt tgacacattg cttgacctga tttatgcaga attaataagc tatttggata    1980
   gtgtagcttt aatgtgctgc acatgatact ggcagcccta gagttcatag atggactttt    2040
   gggacccagc agttttgaaa tgtgtttatg gagtttaaga aatttatttt ccaggtgcag    2100
   cccctgtcta actgaaattt ctcttcacct tgtacacttg acagctgaaa aaaaacaaca    2160
   tgggagtaat aatgggtcaa aatttgcaaa ataaagtact gttttggtgt gggagttgtc    2220
   atgaggctgt gttgaagtga cttatctatg tgggatattg agtatccatt gaaatggatt    2280
   tgttcagcca tttacattaa tgagcattta aatgcaacag atatcatttc aggtgactta    2340
   acatgaatga ataaaagtca atgctattgg aaaaaaaaaa aaaaaa                   2386
<210> 28
   <211> 2594
   <212> DNA
   <213> Homo sapiens
<400> 28

## Description

The present invention relates to the identification of human host factors involved in the early stage of HIV infection. Furthermore, it relates to the use of the identified genes for the elucidation of the mechanism of HIV-infection, as drug targets, and for identifying a compound useful in the treatment of HIV.

### Background of the Invention

A majority of chronic diseases and infection manifest at the integrated level of the cell. Examining disease progression by live cell imaging allows for a high degree of resolution, with the visualization of molecular disease mechanisms and their response to genetic changes. While this type of approach has been very successful in individual experiments, it has remained largely refractory to systematic, genome wide analyses.

During evolution HIV has learned to subcontract a large part of its life cycle to human host factors. In the last decade, several such host factors have been identified through a multiplicity of approaches (for review see ¹). Few of the approaches in the identification of host factors have been systematic, and so far none has been exhaustive. However, the availability of host factors is not only important for the full understanding of the HIV life cycle. Effective ways to mine for viral host factors would further allow the comparison of factor specificity of HIV subtypes with each other or with SIV (Simian immunodeficiency virus), thus pointing to crucial mechanisms in the manifestation of infections, and potential therapy targets.

It was an object of the present invention to identify human host factors involved in the early stage of HIV infection for use in the elucidation of the mechanism of HIV-infection, as drug targets, and for identifying a compound useful in the treatment of HIV.

### Description of the Invention

The objects of the present invention are solved by a nucleic acid having a sequence represented by SEQ ID No. 46, or partial sequences thereof, or sequences complementary to said nucleic acid or to said partial sequences, said partial sequences comprising at least 20 contiguous nucleotides, for use
a) in the elucidation of the mechanism of HIV-infection;
b) as a drug target; or
c) in the identification of a compound useful in the treatment of HIV.

The present invention relates to a method for identifying a compound useful in the treatment of HIV, wherein said method comprises the step of identifying a compound that inhibits activity or expression of the protein encoded by SEQ ID No. 46.

It further relates to a compound inhibiting activity or expression of the protein encoded by SEQ ID No. 46 for use in the treatment of HIV, wherein said compound is selected from siRNAs, ribozymes, and antisense nucleic acids.

WO 2007/141580 discloses the RNASE H2 complex and genes therefor including RNASEH2A.

The term "nucleic acid" as used herein is meant to refer to deoxyribonucleic acid (DNA) and ribonucleic acid (RNA).

The term "sequences complementary to said nucleic acid or to said partial sequences" as used herein also refers to the corresponding (complementary) RNA sequences or partial sequences.

The minimum length of 20 contiguous nucleotides of said partial sequences ensures their specificity. In a preferred embodiment, said partial sequences comprise at least 21, in an even more preferred embodiment at least 22 contiguous nucleotides.

The term "drug" as used herein is meant to refer to a pharmaceutical agent that is suitable for the treatment of HIV.

The initial identification of a compound inhibiting (a) binding to, (b) activity of, or (c) expression of a target protein can be achieved experimentally or be based on available information concerning the target. Compounds inhibiting (a) and (b) are active on the protein level. Compounds inhibiting (c) are directed at the nucleic acids (e.g. DNA, RNA, mRNA) encoding the protein or having a regulatory function. The ability of a compound to bind to a protein can be determined using techniques such as competitive and non-competitive binding assays. Such assays can be performed, for example, using a labeled compound (direct measurement) or detectable reagents that bind to the respective compound (indirect measurement). The encoding nucleic acid sequence of an identified target protein (such as a gene/nucleic acid represented by SEQ ID Nos. 1 to 46, preferably SEQ ID No. 46, 1-2 or 5) provides a target for compounds that are able to hybridize to the nucleic acid. Examples for such compounds include siRNAs, ribozymes, and antisense nucleic acids.

As disclosed herein, a nucleic acid having a sequence represented by any of SEQ ID Nos. 1 to 46, preferably SEQ ID No. 46, 1-2 or 5, or partial sequences thereof, or sequences complementary to said nucleic acid or to said partial sequences, said partial sequences comprising at least 20 contiguous nucleotides, are used as a drug target or in the identification of a compound useful in the treatment of HIV according to the methods outlined in WO 2008/03462228.

A method of identifying a small molecule modulator (which also represents a compound useful in the treatment of HIV) for a target protein candidate as disclosed herein (a protein encoded by any of SEQ ID Nos. 1 to 46, preferably SEQ ID No. 46, 1-2 or 5) may comprise the following steps:
- providing a first cell of a type that is capable of producing a signal when said first cell is exposed to a small molecule modulator, wherein said signal is a signal that can be spatially resolved and, optionally, be quantified, preferably by microscopy,
- exposing said first cell to a small molecule modulator and spatially resolving and, optionally, quantifying a first signal that is produced by said first cell as response to said small molecule modulator,
- providing a second cell of the same type as said first cell,
- performing the following steps:
   - introducing a first nucleic acid encoding a marker protein into a vector,
   - introducing a second nucleic acid encoding said target protein candidate the expression of which is to be detected and/or quantified, into said vector, such that said first and second nucleic acids are operably linked, such that expression of said marker protein is an indication of expression of said target protein candidate,
   - introducing said vector into said second cell,
   - detecting and/or quantifying expression of said marker protein,
   - relating said expression of said marker protein to expression of said target protein candidate, and thereby detecting and/or quantifying expression of said target protein candidate,
- during performance of the above steps on said second cell, after introducing said vector into said second cell, exposing said second cell to said small molecule modulator, and spatially resolving and, optionally, quantifying a second signal that is produced by said second cell as response to said small molecule modulator,
- comparing said first signal with said second signal, and, if there is a difference between said first signal and said second signal, attributing said difference to the expression of said target protein candidate in said second cell, thereby identifying said target protein candidate as a target protein of said small molecule modulator.

Preferably, said first cell and said second cell of the same type as said first cell are LTR-GFP HeLa cells (LTR = long terminal repeat).

A nucleic acid having a sequence represented by any of SEQ ID Nos. 1 to 46, preferably SEQ ID No. 46, 1-2 or 5, or partial sequences thereof, or sequences complementary to said nucleic acid or to said partial sequences, said partial sequences comprising at least 20 contiguous nucleotides, may be used in the elucidation of the mechanism of HIV-infection by utilizing standard methods in the field of network or systems biology, including but not limited to proteomic interaction maps, pull-down experiments (i.e. AP/MS), microarray analysis, and computational modeling²⁹.

SEQ ID Nos. 1 to 46 are listed in **Table 2**.

Through a combination of genome wide RNA interference, high-density cellular microarrays, confocal imaging and image analysis, the inventors have identified so far unknown human host factors involved in the early stage of HIV infection. Using the HIV receptor molecule CD4 as a classifier on 7 visual genome wide analyses, they have shown that HIV-1 employs 0.21 % of the human genome, or 44 host genes, to complete its early live cycle. By combining the high information content of visual images with the rapid reproducibility of array-based genome wide screens, the present approach allowed the identification of HIV-1 host factors with high reliability

To directly identify genes involved in HIV/disease pathogenesis at a cellular level, the inventors have developed a genome wide visual RNA interference approach. It was designed as an automated analysis of pathway and pathogen activity within living cells, using quantitative imaging tools ². Genome wide libraries of short interfering RNAs (siRNAs) and short hairpin RNAs in viral vectors have been screened with low resolution in microtiter plates at a one well per gene level ³. Extending this to high resolution imaging has proven to be very challenging. Cellular microarrays are an alternative, versatile solution for imaging cellular events ^{4,5}. Cell monolayers overlaid on printed spot arrays of siRNA, viral particles or compounds that reverse transfect the cells ⁴⁻¹⁰ provide an alternative to laborious robotic screening ^{4,7,8,10-12}. The inventors have exploited these advantages to screen HIV infection using cellular microarrays.

### Figures

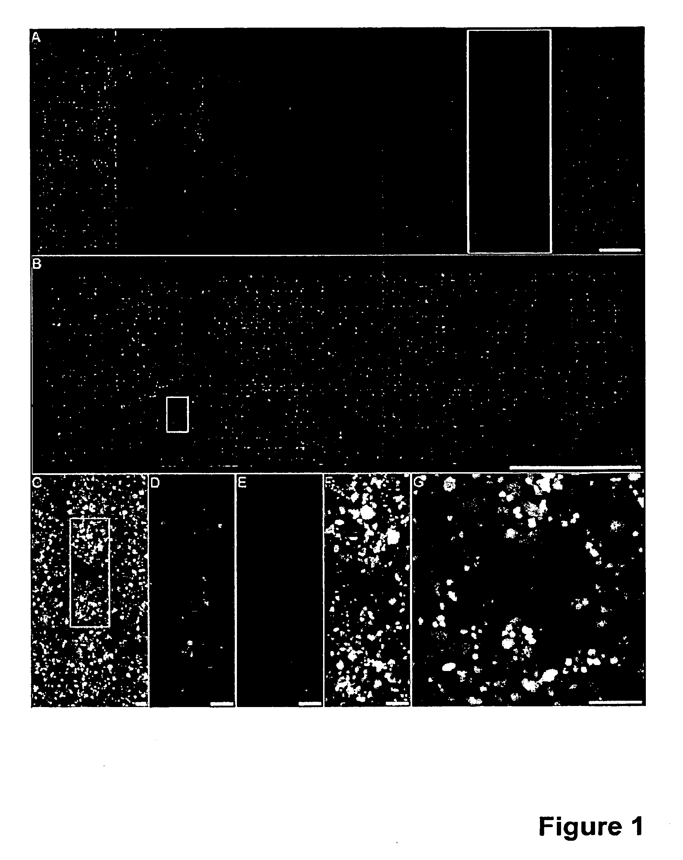

Reference is now made to the figures, wherein
**Figure 1** shows automated genome wide screening of HIV infection in LTR-GFP HeLa cells from genome to single cell using cellular microarrays: Seven cellular microarrays were overlaid with cells for 48 hours, fixed and nuclear stained before three-color automated point scanning confocal imaging. Panel **A** shows an image of a single genome wide screen over seven slides (27,216 spots in total) with 78% (21,127) genome specific siRNAs and 22% (6089) controls. Panel **B** shows a single array from the screen (3,888 spots). Panel **C** shows 18 spots from the single array, three spots from the array where the center spot contained CD4, the upper TRIM50B and lower FLJ43374 siRNAs. Panel **D** shows a spot image, **E** nuclear stain, **F** HIV infection induced GFP expression, and **G** a single spot from the array highlighting CD4 siRNA transfected cells in the spot center and infected control cells bordering the spot. Nuclei were stained with 2.5 µM Draq5 (blue). Scale bars, upper and center panels 10 mm, lower panels 100 µm;

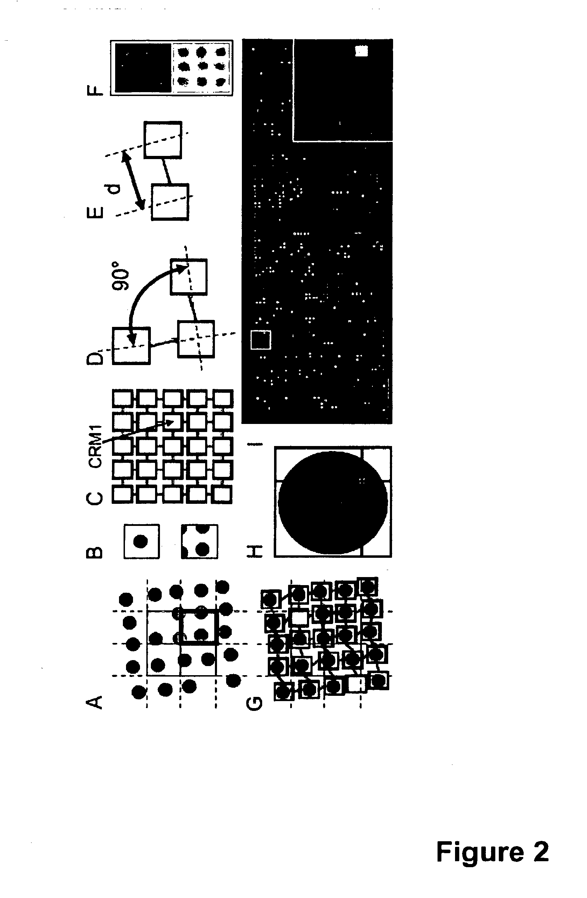

**Figure 2** summarizes the image analysis of the large high resolution cellular micro array: **A** depicts a real array acquisition cartoon showing whole array tilting, missing spots and variations in spot to siRNA spot location (grey dots) relative to image borders (black lines). **B** depicts a hypothetical case with one spot/image (upper panel) compared to the real case with multiple spots/image crossing image borders. **C** shows a flexible array grid model of siRNA annotated nodes (3,888 nodes in total) for fitting to the array miniature. **D** to **F:** the flexible grid function constrained nodes into a relationship with its direct neighbors where **D**) an angle of 90° between two adjacent nodes, **E**) a predetermined spot spacing (d) and **F**) a minimal value on a computed curvature map are favored. **G** shows annotation of the array miniature with the model. **H** depicts a high resolution composite spot image generated from adjacent high resolution images using coordinates from fitting on miniature. **I** shows a fitted large array showing missing spot detection and grid overlay onto red siRNA spot images (inset);

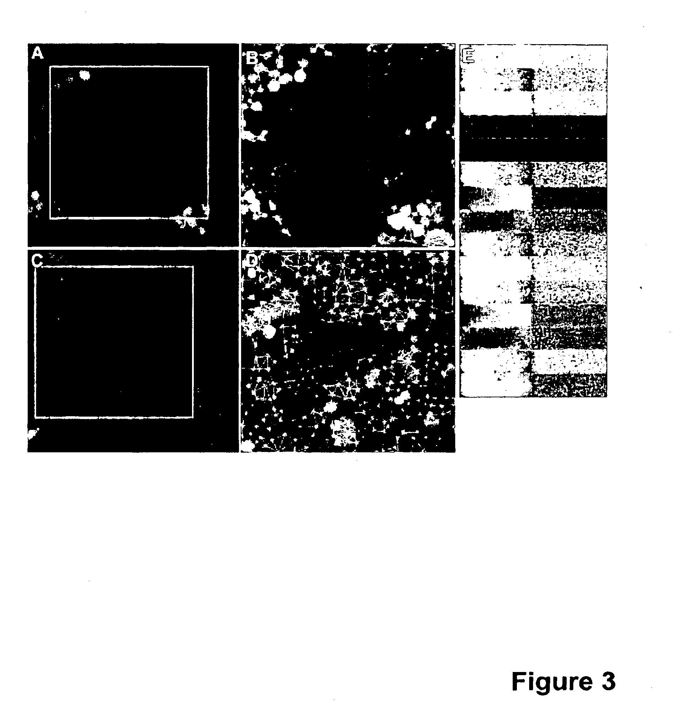

**Figure 3** **A** shows a CD4 siRNA spot image with the spot area automatically detected in the white square. **B** shows CD4 siRNA spot image analysis detecting cell centers inside and outside the spot area to retrieve their number, nuclei intensity, GFP intensity, minimum intensity on the links between cells (the value of the link is the minimum intensity on this link), distance between the cells' intensity and their respective standard deviations along with the total GFP (15 descriptors in total). **C** shows a scrambled siRNA spot image with the spot area detected in the white square. **D** depicts a scrambled siRNA spot image analysis. **E** shows an experiment (=a spot) that is now reduced to a 15-dimensional vector. One array comprises 3,888 spots (108x36 grid). Each result dimension can thus be represented as a 2-D gray level picture with one pixel representing one experiment. The value of each pixel corresponds to the measured dimension. First column: the 15 descriptors measured on the 3,888 spots of a single array. From top to bottom: cellNumber, linkMinGFPAvg, linkMinGFPSdtdev, linkLengthAvg, linkLengthSdtdev, inTotalGFP, inIntNucleiAvg, inIntNudeiSdtdev, inIntGFPAvg, inIntGFPSdtdev, outTotalGFP, outIntNucleiAvg, outIntNucleiSdtdev, outIntGFPAvg, outIntGFPSdtdev. Second column: the same measurements normalized. Those normalized relative values are much more robust to spatial array distortions while conserving local hits;

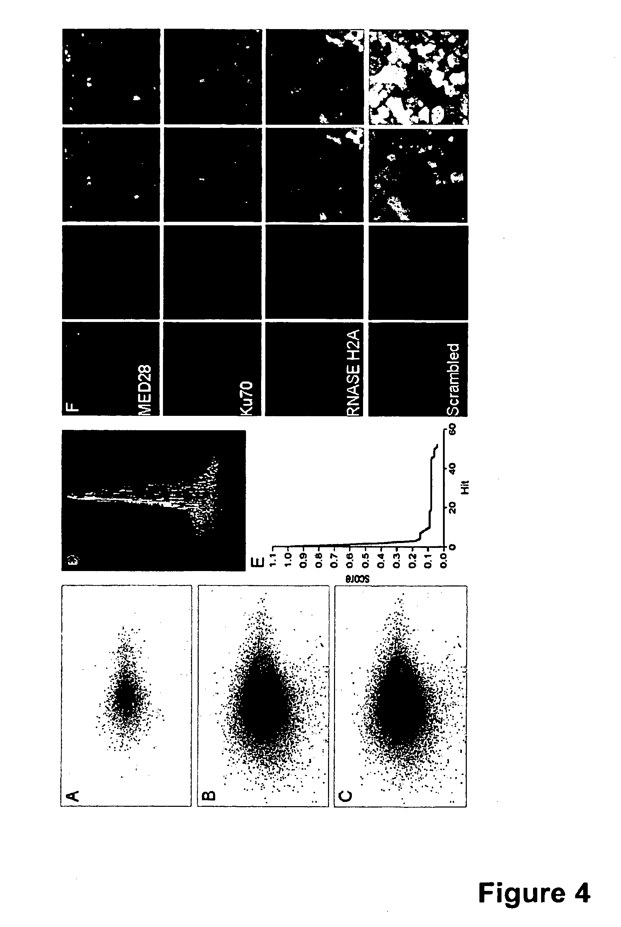

**Figure 4** **A** shows 15-dimensional measurement CD4 and SCRAMBLED clouds projected on the two dimensional space that was the most discriminant between the distributions. One can clearly see that the two classes can be differentiated. **B** shows the projection of the 190,510 experiments (7x7 arrays) onto this same view and **C** shows the classification based on 15-variable results. **A,B,C:** horizontal: canonical axis 1, vertical: canonical axis 2, color: class, intensity value: 15-dimensional Mahalanobis distance (see page 9) to respective class center. **D** depicts the histogram showing the occurrence of a value on the canonical plane (Z axis: the highest occurrence value is 252). Experiments falling in the CD4 class are labeled in green and represent a small fraction of all experiments (0.8%). **E** is a graph showing hits selected among the experiments in the CD4 class which are those with a density ratio under 1 compared to the body of all experiments. Thus, there are proportionally more experiments of the same gene inside the CD4 class than outside. Note that this ratio falls quickly under 0.1 which shows the density of most of the 44 hits is at least 10 times higher inside the CD4 class than outside. **F** shows representative examples of siRNA experiments;

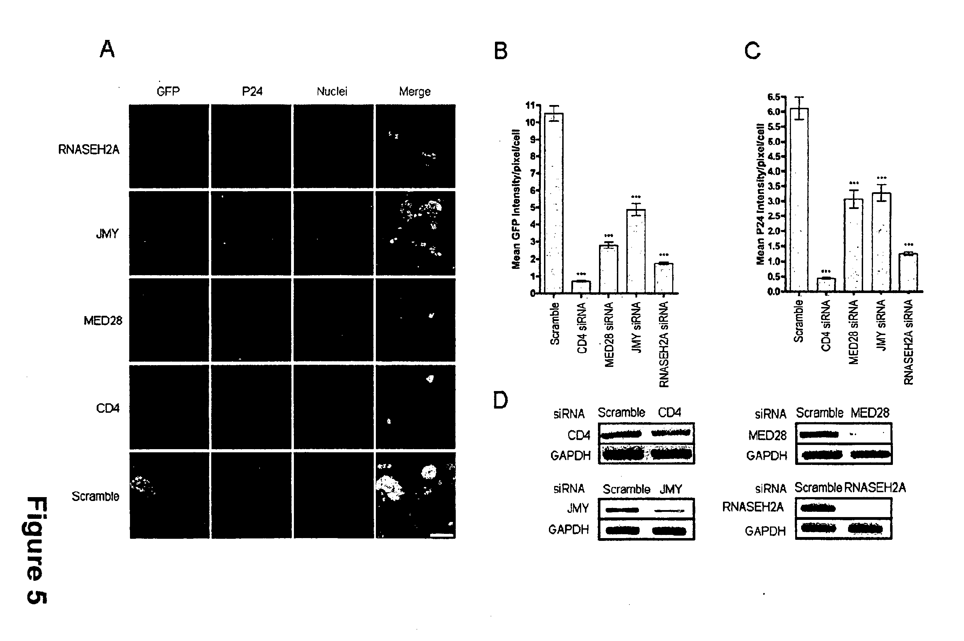

**Figure 5** summarizes the results from LTR-GFP HeLa cells transfected with the indicated siRNA for 24 hours, then infected with HIV-IIIB. After 48 hours, the fixed cells were stained with P24 antibody. **A** shows the block of HIV infection with loss of RNASEH2A, JMY, MED28 and CD4 (positive control). GFP (green) and P24 (red) were used as indicators for HIV infection. Nuclei (blue) were stained with DraQ5. Merge denotes the combined image for nuclei (blue), GFP (green) and HIV P24 (red). Scale bar is 20 µm. **B** illustrates the quantification of GFP expression based on GFP intensity/pixel/cell. **C** illustrates the quantification of P24 expression based on P24 intensity/pixel/cell. **D** shows RNASEH2A, MED28 and JMY mRNA reduction by individual siRNAs. Cells were transfected with the indicated siRNA for 72 hours, then cDNA was prepared and RNASEH2A, MED28 and JMY mRNA expression levels were measured by RT-PCR; and

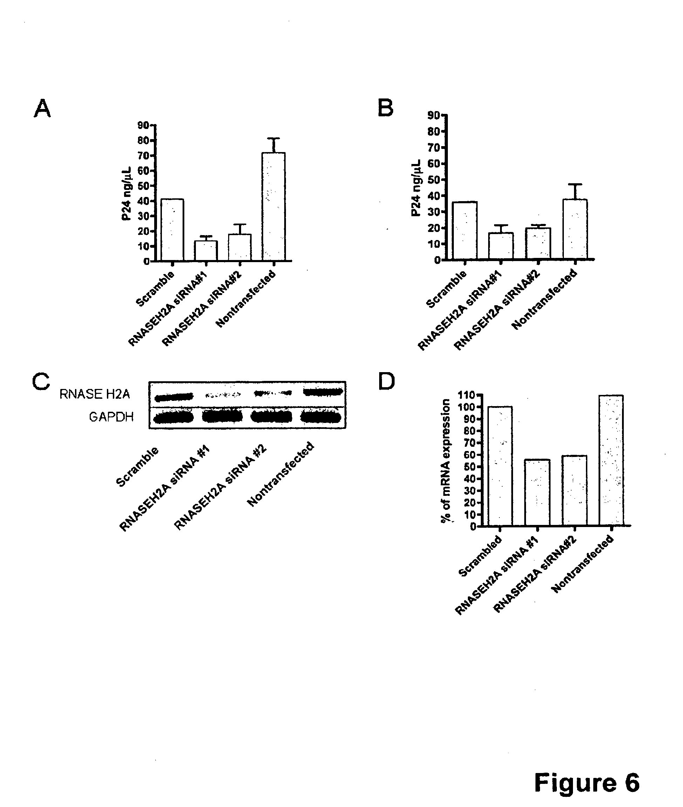

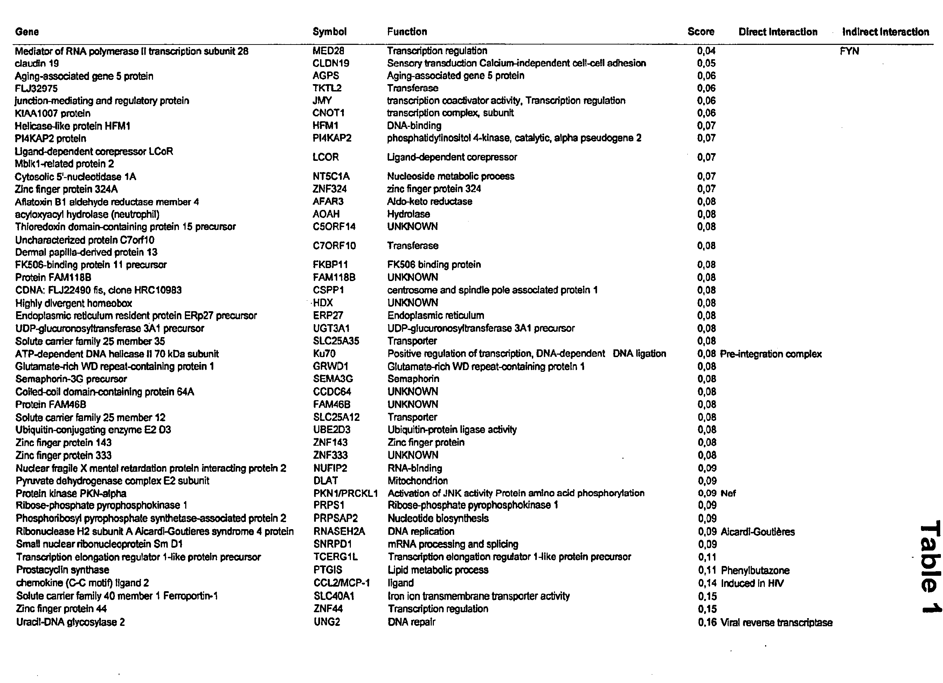

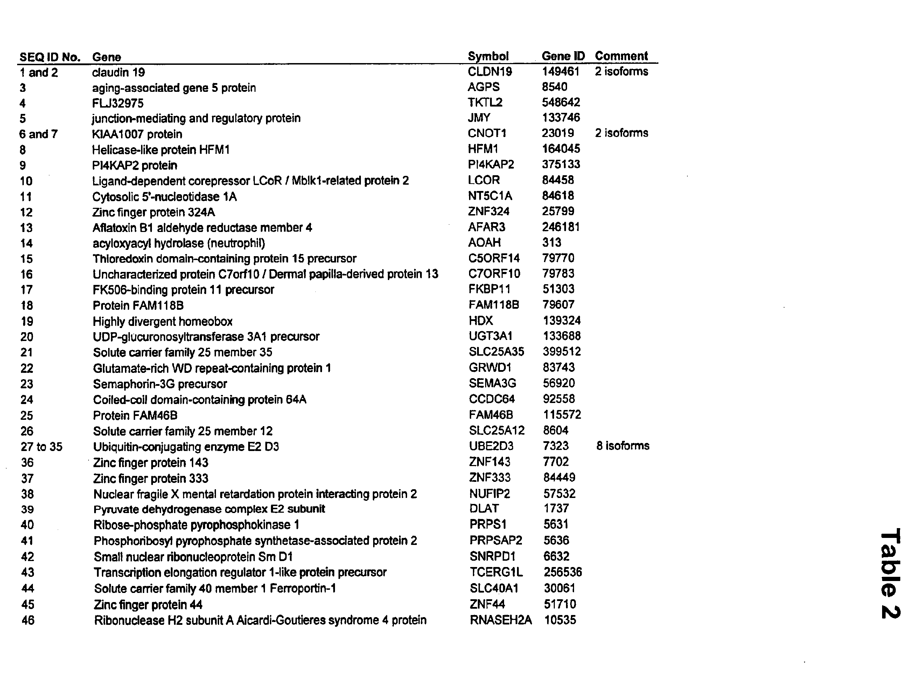

In **Figure 6** **A**, Jurkat cells were transfected with the indicated siRNA for 72 hours, then infected with HIV-1, Strain IIIB virus (MOI: 0.1). After 96 hours, P24 antigen was measured in cell culture supernatants by p24 ELISA. In **B,** Jurkat cells were transfected with the indicated siRNA for 72 hours, then infected with HIV-1, Strain IIIB virus (MOI: 0.05). After 96 hours, P24 antigen was measured in cell culture supernatants by p24 ELISA. C shows RNASE H2A mRNA reduction by individual Acell siRNA. Jurkat cells were transfected with the indicated siRNA for 96 hours, then cDNA was prepared and RNASE H2A mRNA expression levels were measured by RT-PCR. **D** shows the quantification of RNASE H2A knockdown measured by RT-PCR.

### Examples

Cellular microarrays were produced to cover the human genome in a minimal number of arrays, permitting confocal imaging, and removing any dependence on mechanical accuracy for siRNA spot location and imaging ⁷. Arrays printed onto 24 x 60 mm optical glass wafers were mass produced using a high throughput contact printer. Individually bar-coded arrays comprised 3,888 siRNAs as 300µm diameter spots at a pitch of 500 µm in 108 columns x 36 rows. siRNAs were encapsulated in a mixture containing transfection reagent and gelatin ^{7-9,12,13} and red fluorescent siRNA. Red fluorescent siRNA gave optically identifiable individually addressable spots and the entire array could be visualized after cell overlay (**Figure 1**). Reverse transfection was measured 1 to 21 days post-dessication. Using arrays dried for >5 days, close to 60% of the cells were transfected and GFP knock down was >70% 48 hour post-transfection of a GFP expressing HeLa cell line. Endogenous protein expression silencing was >60% 48 hours post-transfection, using indirect immuno-labelling of p65 or exportin 1 (XPO1/CRM_1) and confocal imaging of fixed arrays. Spot to spot contamination was minimal, silencing was low in all neighboring spots (<5%) irrespective of distance from the GFP siRNA spot.

The HIV infection assay comprised 28 hours reverse transfection of HeLa LTR-GFP cells followed by 48 hours of infection with an HIV-1 MOI of 0.14. HIV infection enabled TAT driven expression of the stably integrated GFP and thus recapitulated early steps in viral infection¹⁴. Under these conditions, HIV infection was significantly repressed in cells transfected with a CD4 siRNA (**Figure 1**).

A collection of 84,508 siRNAs, corresponding to four unique siRNA duplexes targeting each of 21,127 unique human genes with control siRNAs, in an encapsulation mixture were printed. Each array comprised 3,888 spots, including 648 controls and the entire human genome was covered in 7 slides **(****Figure** 1**A**). Arrays were imaged using an inverted automated point scanning confocal microscope. It acquired an entire array in 1,820 800 µm sided three-color 16 bit images, equivalent to ~ 10 gigabytes of image information for an array covering 1/7th of the genome (70 gigabytes per genome). 7 genomes were acquired in total (7x7 arrays = 49 arrays) encompassing almost two hundred thousand individual experiments and half a terabyte of imaging data.

A single visual genome wide analysis for HIV infection is shown in **Figure 1****.** It spans from 7 arrays containing the human genome siRNA library (**Figure 1A**), to a single array (**Figure 1B**) and systematically from that array to a single spot where HIV infection was repressed after CD4 silencing **(****Figure 1C** to **G****).** The image resolution was suitable for high content morphological and phenotypic analysis ¹⁵, however the size and topology of the array image datasets necessitated novel image analysis solutions.

A first image analysis goal was to identify and annotate spots on the arrays. Here, there is no relationship between the printed array, siRNA annotation and image sequence (**Figure 2A**). Furthermore, the quantity of images is significant (5,460 for one array in three colors). Single array images contained up to four spots - often shared with neighboring images - at 20x resolution (**Figure 2B**), with no intrinsic information on spot identity. Inherent mechanical and positioning inaccuracies in array printing produced arrays tilted and displaced in XY as a whole and there were spot to spot variations in relative row/column spacing, spot positioning and alignment (**Figure 2A**). Importantly, the uncompressed images of the array were so large that if assembled into one contiguous image, the image could not be viewed or manipulated because of limitations in processing power. Dedicated software automatically gathered individual images and created a compressed mosaic miniature retaining the spatial information in the high resolution images stored on the server. This image was 2,500-fold compressed compared to the original (4 Mb vs. 10 Gb). Fitting the array used the image miniature to annotate the spots with their siRNA identity. It was achieved by minimizing a function of the 3,888 x-y variables (3,888 spot locations). This used two constraints: the first was a flexible annotated grid model (model constraint) which had a high value when the flexible grid was severely distorted from the original grid model. This was achieved by controlling the angle between adjacent nodes (**Figure 2D**) and the known inter-spot distance (**Figure 2E**), and the value progressively increased with these distortions. The second constraint was a transform of the miniature (image constraint) where the value decreased as the center of the spot was approached. This was achieved by computing a curvature map of the miniature (**Figure 2F**). The algorithm defined a rough approximation and then minimized the above function to make the nodes of the grid model fit with the spots of the miniature (**Figure 2G**). Each spot was then annotated with the siRNA identity and reconstructed in real time from the raw 16 bit images from the database, using the back transform of the spot locations determined by the fitting (**Figure 2H**). These high resolution images of the spots were then used for image analysis (**Figure 2I**).

Seven complete human genomes, a total of 49 siRNA arrays, were cultivated for 28 hours to permit host gene silencing. Arrays were infected with live HIV-1 at a multiplicity of infection of 0.14 for 3 hours, washed and incubated for 45 h prior to imaging. Once the grids were fitted and the identity of each spot retrieved, HIV infection was independently analyzed on each spot using the following image and data analysis strategy. Measurement of GFP fluorescence alone would yield no information on cell shape, density and cell:cell fusion. More importantly, dilution of the GFP signal during syncitial formation can not easily be distinguished from repression of GFP production. To address this, the inventors developed an algorithm that retrieved 15 descriptors.

To be able to quantify HIV infection, the inventors measured cells within the siRNA spot and in a border around the spot. This was achieved using an algorithm that randomly measured pixels in an image greater than the spot to define the best possible spot location (Figure 3A, white square) given the predicted spot dimensions. Nuclear centers were detected by template matching of a two dimensional Gaussian shape which roughly modeled a stained fluorescent nucleus in the images. GFP and nuclear staining was measured on a small disk located on those nuclear centers. This measurement was robust relative to background or cell shape variations. To measure syncitia formation and cell dispersion, a Delaunay triangulation¹⁶ was computed from the set of all nucleus centers to establish a unique neighborhood map. The minimum value of GFP for each unique nucleus: nucleus link was retrieved and the Delaunay triangulation gave the Voronoi diagram¹⁷ which was used to separate densely-packed nuclei. The algorithm produced a 15-dimensional vector for each experiment/spot within 150 ms (cellNumber, linkMinGFPAvg, linkMinGFPSdtdev, linkLengthAvg, linkLengthSdtdev, inTotalGFP, inIntNucleiAvg, inIntNucleiSdtdev, inIntGFPAvg, inIntGFPSdtdev, outTotalGFP, outIntNucleiAvg, outIntNucleiSdtdev, outIntGFPAvg, outIntGFPSdtdev). **Figure 3B** shows the visual result of applying the algorithm to a CD4 spot and a SCRAMBLED spot.

Given the variations in cell culture, the measurements of infection were normalized across the arrays. The array is a large experiment with a low frequency spatial variation due to imperfect cell density across its surface. Array images representing each dimension of the result vector for a whole single array are shown in **Figure 4A**. A median filter with a radius size of 3 spots (defined as the radius which maximizes the separation between controls classes, see below) was used to filter result arrays. This normalization produced relative value that were comparable for all measurements across the 7 genomes.

The inventors' intent was to identify genes that were as potent in repressing early stage HIV infection as the control CD4. Thus, they built a two class classifier from two control distributions of 215 individual CD4 and 3896 SCRAMBLED experiments. Since they were both 15-variate Gaussian distributions, they set a simple and robust classifier and computed the most discriminating projection as shown in Figure 4B**-E****.** The inventors computed the 15-dimensional Mahalanobis distance relative to CD4 and SCRAMBLED classes for all 190,510 data points. The Mahalanobis distance is a distance measure introduced by P. C. Mahalanobis in 1936. It is based on correlations between variables by which different patterns can be identified and analyzed. It differs from Euclidean distance in that it takes into account the correlations of the data set. The inventors selected only the points closer to CD4 than SCRAMBLED and simultaneously with a distance to the CD4 class center inferior to the square root of a given g which is determined from a chi-squared (with 15 degrees of freedom) as corresponding to a gating probability chosen to be at least 0.99 (a point has to be in the CD4 class with a probability of 0.99, excluding malformed and unrelated experiments nevertheless closer to CD4 than to SCRAMBLED). This identified 1680 experiments (0.8%) as potentially similar to CD4. The inventors computed a score ratio based on experiment density in both classes. For each gene, the ratio between the percentages of experiments inside CD4 class versus outside was computed. All genes under a score of 1 were considered to have an abnormally high representation in the CD4 class and the lower the score the stronger that representation. For example CD4 has a value of 0.043 which means it is 23 times (1/0.043) denser in CD4 class than outside. 44 genes were identified which had a ratio under 0.1 demonstrating their over representation in the CD4 class (**Table 1,** **Figure 4**). A score of lower than 0.1 means a density at least ten times higher inside the CD4 class than outside (**Figure 4**).
Of these 44 genes, 36 were identified as novel in terms of their involvement in HIV infection (see **Table 2**). The remaining eight genes have been previously shown, either directly or indirectly, to be involved in HIV infection. Recently, a functional genomic screen by Brass et al. ¹⁸ revealed MED28, CSPP1 and ERP27 as three of several host genes which encode for proteins required for HIV infection. Prior to this, MED28 (magicin) had already been identified as an interacting partner of FYN ¹⁹, a known HIV interacting partner. Ku70 is a well known mediator of the early steps of retroviral infection due to its interaction with retroviral replication intermediates and pre-integration complexes ²⁰. PKN1 (also known as Pak1) has been shown to interact with the HIV accessory factor Nef, and depletion of PKN1 strongly inhibited HIV infection in multiple cell systems ²¹. The FDA-approved drug phenylbutazone, which targets PTGIS, has been patented as a potential antiviral (including HIV) agent for both humans and animals ²². CCL2/MCP-1 codes for a proinflammatory chemokine which is induced by the HIV matrix protein p17 during HIV infection ²³, ²⁴. Furthermore, it has previously been reported that UNG2 is packaged into HIV viral particles and physically associates with the viral reverse transcriptase enzyme ²⁵. The fact that over 20% of the identified genes are known effectors of HIV infection validates the overall results presented herein. Interestingly, mutations in the RNASEH2A gene have been shown to result in the neurological disorder Aicardi-Goutières syndrome (AGS) ²⁶.

To verify the significance of the screening results, it is essential to prove that, ultimately, depletion of the candidate genes in LTR-GFP HeLa cell blocks HIV replication in a way similar to that seen by CD4 knockdown. To test this, we selected RNASEH2A, MED28 and JMY and used CD4 as a control. Cells were transfected with individual siRNA for 24 hours and were infected with HIV for 48 hours. Viral replication was measured by following the appearance of GFP and P24 expressing cells. ¹⁴ RNASEH2A, MED28 and JMY knockdown in cells blocked HIV infection (**Figure 5A**). Quantification of these images was done using GFP intensity and P24 expression (**Figure 5B, 5C**). RNASEH2A knockdown resulted in almost 80% reduction in HIV infection compared with the scramble siRNA transfected population (**Figure 5B, 5C**). Also MED28 and JMY knockdown resulted in approximately 50% reduction in HIV infection (**Figure 5B, 5C**). We also verified that the targeted mRNAs were down-regulated by measuring their expression by RT-PCR (**Figure** 5**D**). Therefore, RNAseH2A, MED28 and JMY are necessary for the HIV infection process.

To further verify the significance of the screening results, RNASEH2A was used as a representative gene for knockdown in a more representative cell type for HIV infection. For effective gene silencing, individual RNASEH2A #1 and #2 siRNAs were transfected into Jurkat (T lymphocyte) cells. It was verified that the targeted mRNA was down-regulated by its expression by RT-PCR both in a qualitative (**Figure 6C**) and quantitative fashion (**Figure 6D**). About 50% RNASEH2A was silenced. RNASEH2A siRNA transfected cells were infected with HIV at two different MOIs, and viral replication was measured by P24 ELISA (**Figure 6A****, B**). In summary, knock down of RNASEH2A abrogates the replication of WT HIV1 virus in Jurkat cells. These observations confirm the previous findings that RNASEH2A is required for the HIV infection process.

### Materials and Methods

### Chemicals

All fine chemicals were purchased from Sigma-Aldrich. DRAQ5 was from BioStatus (Shepshed, UK). All siRNA duplexes were purchased from Dharmacon (USA). The siRNA library comprised 1.0 nM of the Dharmacon siARRAY whole human genome siRNA library (Thermofisher, West Lafayette, CO) containing to 84,508 siRNAs corresponding to four unique siRNA duplexes, targeting each of 21,127 unique human genes. Primary antibodies were from Santa Cruz Biotechnology, and all fluorescent secondary antibodies were from Molecular Probes/Invitrogen (Carlsbad, CA). Transfection reagents were from commercial sources.

### Cell lines and cell culture

LTR-GFP HeLa cells (A. Boese, Institut Pasteur Korea) were produced as described ¹⁴ wild type HeLa (ATCC) and GFP-torsin expressing HeLa (R. Grailhe, Institut Pasteur Korea) were cultivated in high glucose glutamax Dulbecco's modified eagles medium (Invitrogen; Carlsbad, USA) supplemented with 110mg/mL sodium pyruvate, 10% fetal calf serum (Gibco, USA) and 1% penicillin streptomycin (Invitrogen; Carlsbad, USA). Stable lines were maintained in medium supplemented with selection marker. Cell lines were cultivated on arrays for 12 to 72 hours for quantifying reverse transfection. For HIV infection, 650,000 cells were seeded per array (24x60 mm) and cultivated in Opti-MEM (Invitrogen; Carlsbad, USA) supplemented with 5% fetal calf serum (Gibco, USA) and 1% penicillin streptomycin (Invitrogen; Carlsbad, USA) for 28 hours. Cells were inoculated overnight with HIV-1 strain IIIB (Daymoon industries; Cerritos, USA) at an MOI of 0.14. Fresh Opti-MEM (Invitrogen; Carlsbad, USA) supplemented with 5% fetal calf serum (Gibco, USA) and 1% penicillin streptomycin (Invitrogen; Carlsbad, USA) was added the following day. Cells were cultivated for an additional 45 hours, followed by fixation in 1% (w/v) paraformaldehyde in Dulbecco's phosphate buffered saline, and nuclei were stained with 2.5 µM Draq5 (BioStatus, UK) before imaging. Jurkat clone E6-1 (ATTCC) was cultured in RPMI medium 1640 (Invitrogen; Carlsbad, USA) supplemented with 10% Fetal Calf Serum (Gibco, USA), 1% penicillin streptomycin (Invitrogen; Carlsbad, USA), 1mM sodium pyruvate (Gibco, USA), 10 mM HEPES.

### Microarray printing

siRNA transfection solution was prepared essentially as described ²⁷ and printed as 3,888 spot arrays (108 x 36 spots) on No. 1 glass coverslips using stealth pins (telechem, USA) and a high throughput microarray printer (Genomic Solutions, USA) at 22-25°C, 55-65% RH enclosed in a custom built clean chamber providing a sterile HEPA filtered atmosphere. Arrays were stored in a dessicator with no significant alternations in performance from 1 week to 8 months post-printing. 7 slides covered the genome and contained 16% of control siRNA spots.

### Microarray acquisition and analysis

Arrays were acquired with a point scanning confocal reader (Imageexpress Ultra, Molecular Devices, USA) as 16 bit TIFF files written directly to an external database. Images were read directly from the database for analysis using software designed for this purpose. Adaptive gridfitting was applied to identify siRNA spots in the entire array, fit the spots and crop them before extracting the image data for analysis, annotation and result export.

### RNA isolation and RT-PCR

Total RNA was isolated from siRNA transfected LTR-GFP HeLa and/or Jurkat cells by Trizol method (Invitrogen, USA). cDNA was made using 1 µg of total RNA and MMLV-reverse-transcriptase (Promega) in a 25 µl reaction mixture in the presence of 50 pmol oligo(dT) primer and 20 µM dNTP mixture for 60 min at 37°C. For PCR amplification, specific oligonucleotide primer pairs (0.2 pmol each) were incubated with 200 ng cDNA, 1 unit of LA Tag polymerase (Takara), 1X LA PCR buffer 2 (2.5 mM MgCl₂) and 100 µM dNTP in a 25 µL reaction mixture. The sequences of primers used are as follows:
RNASEH2A sense primer 5'- GACCCTATTGGAGAGCGAGC-3' and RNASEH2A antisense primer 5'-GTCTCTGGCATCCCTACGGT-3';
JMY sense primer 5'- GCAACTCTGGTTAGGAGCCC-3' and JMY antisense primer 5'- TATCTCCTCGGAGACCGTCC-3';
MED28 sense primer 5'- GGACTATGTCAATGGCACCG-3' and MED28 antisense primer 5'-TTGTGCTGCACGTTGATGTC-3';
CD4 sense primer 5'- GGATAGTGGCACCTGGACAT-3' and CD4 antisense primer 5'-CTTGCCCATCTGGAGCTTAG-3'; and
GAPDH sense primer 5'- TGATGACATCAAGAAGGTGGTGAAG -3' and GAPDH antisense primer 5'-TCCTTGGAGGCCATGTGGGCCAT-3'.
PCR conditions were 95°C for 30 sec, 54°C for 30 sec, and 72°C for 3 min, for a total of 40 cycles. The PCR products were applied onto a 1% agarose gel and visualized with Ethidium bromide.

### Forward siRNA transfection and virus infection

Jurkat cells (40,000 cells/ well) were transfected with 1 µM Acell siRNA (Dharmacon, USA) against selected individual RNASEH2A#1, #2 or scrambled in 24-well plates, and then incubated for 72 hours. Cells were infected with HIV-1, Strain IIIB virus (Daymoon industries; Cerritos, USA) MOI 0.5, 0.01 and inoculated for 3 hours. After viral supernatant was removed, cells were cultivated in RPMI medium 1640 (Invitrogen; Carlsbad, USA) supplemented with 10% Fetal Calf Serum (Gibco, USA), 1% penicillin streptomycin (Invitrogen; Carlsbad, USA), 1 mM sodium pyruvate (Gibco, USA) and 10 mM HEPES for 96 hours. Virus replication was determined by detection of p24 HIV-1 viral core antigen in cell-free supernatants by a P24 ELISA (Perkin-Elmer).

LTR-GFP HeLa cells (5,000 cells/well) were transfected with 50 nM of a selected siRNA (Dharmacon, USA) in 96-well plates followed by incubation for 24 hours. Cells were infected with HIV-1, Strain IIIB virus (Daymoon industries; Cerritos, USA) and inoculated for 3 hours. After viral supernatant was removed, cells were cultivated in growth medium. Cells were fixed in 4% (w/v) paraformaldehyde in Dulbecco's phosphate buffered saline, stained with Anti-P24 antibody (Abcam, USA) and stained with 2.5 µM Draq5 (Biostatus, UK) before imaging.

### P24 immunofluorescence detection

Cells were washed twice with phosphate-buffered saline (PBS), fixed for 10 min with 4% (w/v) paraformaldehyde in PBS and then washed with PBS. For permeabilization, cells were incubated in 0.1% Triton-X 100 in PBS for 10 min and subsequently washed in PBS. Then followed incubation with a 1:200 dilution of mouse anti-P24 antibody in 10% goat serum in PBS for 2 hours at 4°C. Plates were washed 3 times with PBS for 10 min on an orbital rotator. Alexa 532 goat anti-mouse secondary antibody (1:1000) was incubated with the cells for 1 hour at room temperature and cells were then washed 3 times for 10 min with PBS on an orbital shaker before the addition of 5 µM of DraQ5 in PBS for 10 min at room temperature.

### References

¹ J. Lama and V. Planelles, *Retrovirology* **4**, 52 (2007).
² S. G. Megason and S. E. Fraser, Cell 130 (5), 784 (2007).
³ A. W. Whitehurst, B. O. Bodemann, J. Cardenas et al., Nature 446 (7137), 815 (2007).
⁴ R. Z. Wu, S. N. Bailey, and D. M. Sabatini, Trends in Cell Biology 12 (10), 485 (2002).
⁵ J. Ziauddin and D. M. Sabatini, Nature 411 (6833), 107 (2001).
⁶ S. N. Bailey, S. M. Ali, A. E. Carpenter et al., Nature Methods 3 (2), 117 (2006); S. N. Bailey, D. M. Sabatini, and B. R. Stockwell, Proceedings of the National Academy of Sciences of the United States of America 101 (46), 16144 (2004); D. B. Wheeler, S. N. Bailey, D. A. Guertin et al., Nature Methods 1 (2), 127 (2004).
⁷ H. Erfle, B. Neumann, U. Liebel et al., Nature Protocols 2 (2), 392 (2007).
⁸ H. Erfle, J. C. Simpson, P. I. Bastiaens et al., BioTechniques 37 (3), 454 (2004).
⁹ J. C. Simpson, C. Cetin, H. Erfle et al., Journal of Biotechnology 129 (2), 352 (2007).
¹⁰ D. B. Wheeler, A. E. Carpenter, and D. M. Sabatini, Nature Genetics 37 Suppl, S25 (2005).
¹¹ C. Conrad, H. Erfle, P. Wamat et al., Genome Research 14 (6), 1130 (2004); U. Liebel, V. Starkuviene, H. Erfle et al., FEBS Letters 554 (3), 394 (2003); B. Neumann, M. Held, U. Liebel et al., Nature Methods 3 (5), 385 (2006).
¹² H. Erfle and R. Pepperkok, Methods in Enzymology 404, 1 (2005).
¹³ H. Erfle and R. Pepperkok, Methods in Molecular Biology (Clifton, N.J 360, 155 (2007).
¹⁴ D. I. Dorsky, M. Wells, and R. D. Harrington, J Acquir Immune Defic Syndr Hum Retrovirol 13 (4), 308 (1996).
¹⁵ C. Bakal, J. Aach, G. Church et al., Science (New York, N.Y 316 (5832), 1753 (2007).
¹⁶ B. Delaunay, Izvestia Akademii Nauk SSSR Otdelenie Matematicheskikh i Estestvennykh Nauk 7, 793 (1934).
¹⁷ G.F. Voronoi, J. Reine Angew. Math. 134, 198 (1908).
¹⁸ A. Brass, D. Dykxhoorn, Y. Benita et al., Science 319 (5865), 921 (2008).
¹⁹ M-F. Lee, R. Beauchamp, K. Beyer et al., Biochem Biophys Res Commun. 348, 826 (2006).
²⁰ L. Li, J. M. Olvera, K. E. Yoder et al., The EMBO Journal 20 (12), 3272 (2001).
²¹ D. Nguyen, K Wolff, H. Yin et al., J Virol. 80 (1), 130 (2006).
²² J-O. Miesch, US04956377.
²³ E. Marlnl, L. Tlberlo, S. Caracclolo et al., Cell Microbiol. 10 (3), 655 (2008).
²⁴ A. Ansari, R. Schmidt and H. Heiken, Clin Immunol. 125, 1 (2007).
²⁵ S. Priet, J-M. Navarro, N. Gros et al., J Biol Chem. 278 (7), 4566 (2003).
²⁶ Y. Crow, A. Leitch, B. Hayward et al., Nat Genet. 38 (8), 910 (2006).
²⁷ H. Erfle, B. Neumann, U. Liebel et al., Nature Protocols 2 (2), 392 (2007); H. Erfle and R. Pepperkok, Methods in Enzymology 404, 1 (2005).
²⁸ N. Emans and U. Nehrbass, WO 2008/034622.
²⁹ G. Ilsley, N. Luscombe and R. Apweiler, Biochim Biophys Acta doi:10.1016/j.bbapap.2009.05.002 (2009).

## Claims

1. Method for identifying a compound useful in the treatment of HIV, wherein said method comprises the step of identifying a compound that inhibits, activity or expression of the protein encoded by SEQ ID No. 46.

2. Compound inhibiting activity or expression of the protein encoded by SEQ ID No. 46 for use in the treatment of HIV, wherein said compound is selected from siRNAs, ribozymes, and antisense nucleic acids.

## Patentansprüche

1. Verfahren zum Identifizieren einer Verbindung, die zur Behandlung von HIV nützlich ist, wobei das Verfahren den Schritt des Identifizierens einer Verbindung umfasst, die die Aktivität oder Expression des durch SEQ ID NO: 46 kodierten Proteins inhibiert.

2. Verbindung, welche die Aktivität oder Expression des durch SEQ ID NO: 46 kodierten Proteins inhibiert, zur Verwendung bei der Behandlung von HIV, wobei die Verbindung ausgewählt ist aus siRNAs, Ribozymen und Antisense-Nukleinsäuren.

## Revendications

1. Procédé pour identifier un composé utile pour le traitement du VIH, dans lequel ledit procédé comprend l'étape d'identification d'un composé qui inhibe l'activité ou l'expression de la protéine codée par SEQ ID No.46.

2. Composé inhibant l'activité ou l'expression de la protéine codée par SEQ ID No.46 pour l'utilisation dans le traitement du VIH, dans lequel ledit composé est choisi parmi le groupe comprenant les ARNsi, les ribozymes et les acides nucléiques antisens.
